# EUROPEAN PATENT SPECIFICATION

(11) **EP 3 416 563 B1**
(45) Date of publication and mention of the grant of the patent: **05.05.2021**
(21) Application number: 17753864.2
(22) Date of filing: 16.02.2017
(51) Int. Cl.: A61B 6/03, A61B 6/14, A61C 8/00, A61C 1/08

(54) **SEQUENTIAL DENTAL SURGICAL GUIDE SYSTEM**
DENTALES SEQUENTIELLES CHIRURGISCHES FÜHRUNGSSYSTEM
SYSTÈME DE GUIDE CHIRURGICAL DENTAIRE SÉQUENTIEL

(30) Priority: 16.02.2016 US 201615044979
(43) Date of publication of application: 26.12.2018
(73) Proprietor: Watson, Jason, La Crescenta, CA 91214 (US); Institut Straumann AG, 4002 Basel (CH)
(72) Inventor: WATSON Jason, La Crescenta, CA 91214 (US)
(74) Representative: Michalski Hüttermann & Partner Patentanwälte mbB
(86) International application number: PCT/US2017/018239
(87) International publication number: WO 2017/143107

(56) References cited:
- US-A- 5 246 370
- US-A1- 2010 183 998
- US-A1- 2014 099 600
- US-A1- 2014 099 600
- US-A1- 2015 010 881
- US-A1- 2015 272 704
- US-A1- 2016 278 878
- US-B2- 8 529 255

## Description

### Technical Field

The present invention relates to the field of dental surgery. More particularly, the present invention relates to a sequential dental surgical guide system that employs a set of anchoring sites that occupy the same relative positions on each guide such that each of the guides are fixed in the same set of osteotomies. The sequential dental surgical guide system may also include features that provide a template or boundary for reducing j aw bone.

### Background Ar

A document in the art is exemplified by US 2014/099600 A1. It discloses a system comprising a first, a second and a third dental surgical guide, wherein the first and the second surgical guides comprise anchoring sites for attachment to the jaw bone of the patient. The anchoring sites occupy the same relative positions on the first and second dental surgical guides such that they can be aligned and secured by a single set of osteotomies determined by the anchoring sites.

The use of dental prostheses with dental implants secured in the upper or lower jawbone is well known in the art. Accurate placement of the implants within the jawbone is a difficult task. The dental surgeon typically has difficulty deciding on a drilling axis for the implants since the ideal position for the implants should be decided with knowledge of the jawbone structure into which the implant is to be inserted, and with knowledge of the position within the jawbone structure of the nerve tissue, the gum surface and the required position and dimensions of the false teeth or dentures to be supported by the dental implant.

The conventional surgical procedure for installing one, implant-supported, prosthetic tooth includes drilling a properly positioned hole in the jawbone of the patient, inserting the implant in the hole, and attaching the prosthetic tooth to the implant. Proper implant positioning is also extremely important to ensure that the implant is anchored within sufficient bone structure in the patient's jawbone.

The most common method for locating a dental implant hole is to visually survey the area and drill the hole in a freehand manner. However, this method can readily result in imperfect bores due to space limitations associated with working inside a patient's mouth. If the drilling axis is not properly chosen, the installed implants might cause damage to the tissues and muscle surrounding the area of implantation and subsequently cause temporary or permanent paresthesia. Furthermore, other problems can result from flawed or imperfect implant holes, such as uneven force distribution, insufficient bone growth around the implant, secondary infections, and ultimately, implant failure.

During a one-stage surgical procedure, a healing abutment assists in the healing, formation, and maintenance of the soft tissue over the implant while bone integration occurs. The healing abutment is immediately placed into the implant well to ensure that the gum line will heal properly and look natural once the final abutment is tapped into the implant.

During a two-stage surgical procedure, a cover screw is initially screwed into the implant well. The tissue surrounding the cover screw is then sutured to protect the implant site while bone integration occurs. Once the bone has integrated, an incision is made in the tissue above the site, at which time integration is checked. Once the bone has healed properly, the cover screw is then removed, and a healing abutment is screwed into the implant well. With the healing abutment securely in place the tissue surrounding the area is sutured. Approximately 4-6 weeks later, the healing abutment can be removed and a final abutment is then placed into the implant. In some surgical cases, a final abutment is immediately placed into the implant well rather than the healing abutment and sutures are not required. In both the one-stage and two-stage surgical procedure, the final abutment supports the final crown or denture.

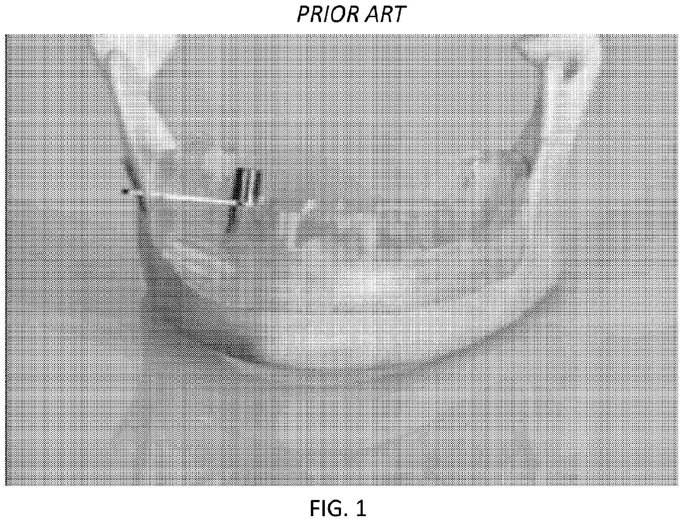

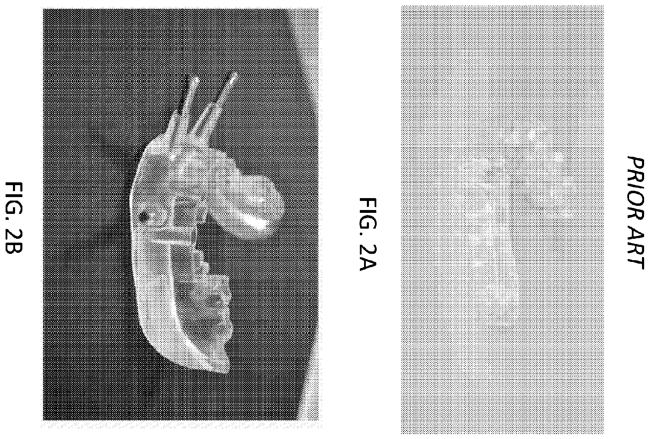

Many types of surgical guides are on the market today. They can be tooth supported, gum supported, or bone supported. The simplest guides are done in the laboratory. They consist of acrylic templates (or stents), or teeth, both filled with radiopaque markers that provide the position of the teeth in relation to the bone on 2D radiographs. Holes are drilled through these surgical guides at the selected implant sites and the surgeon uses them to make bone perforations. Afterwards, the surgeon needs to raise a flap in order to make the osteotomies. Examples of such guides are shown in FIGS. 1 and FIGS. 2A-2B. More sophisticated guides use computer tomography (CT)-scan data and special software in order to place the implants according to three-dimensional (3D) data. The guide is then fabricated using stereolithographic machines or milling machines.

Each surgical stent commonly used is custom-built and these devices are only useful for a single patient, are costly to fabricate, and they require a number of intermediary office and laboratory steps to take an impression of the patient's arch and create a cast model from which the surgical stent is formed.

Superstructures are used as load-bearing elements that interface prosthesis to implants. In the conventional method for the construction of superstructures, a physical model of the patient's gums and dental implant heads is prepared on which the superstructure is built manually using molding and other techniques known in the art. The craftsman or technician skilled at manufacturing such dental superstructures takes into consideration the size and shape of the desired dentures to be placed over the superstructure when crafting the same. The procedure for manufacturing dental implant superstructures as is conventionally known in the art is time-consuming and sometimes results in imperfect structures or defects in the visual appearance of the dentures to be placed over the superstructure.

Therefore, in an effort to reduce costs and the number of steps associated with fabricating a traditional surgical stent, various forms of prefabricated surgical stents and positioning guide systems have been developed to aid the dental surgeon. In International patent application publication no. WO 94/26200, there is described an adjustable guiding device for positioning dental implants in which it is possible for the dental surgeon to adjust a drilling axis for each implant before proceeding to use the guiding device or drill template to guide the surgeon's drill for the purposes of preparing the drill hole for the implant.

In U.S. Pat. No. 5,401,170, there is disclosed a method and apparatus for measuring by camera image the implant heads of the implants in the patient's mouth for the purposes of cutting a frame on which the prosthetic teeth will be arranged and baked. In the method disclosed, the construction of the frame or superstructure is carried out in the absence of a reference to the shape and position of the patient's ideal tooth position.

Thus, as the dentures or artificial teeth are crafted on the frame or superstructure, care would be required during the manual process to ensure that the position of the teeth on the frame will match the opposed set of teeth in the patient's mouth.

It would thus be desirable to provide a drill guide system comprising components fabricated prior to the actual surgery that may be used more than once for the same patient, for any restoration configuration, and that enables precise implant spacing, and also ensures that the implant holes are drilled at the proper angle and orientation.

### Disclosure of the Invention

One embodiment of the invention comprises a dental device comprising a surgical template having one or more sites for drilling osteotomies in the maxillary or mandibular jaw and a false teeth set. For such dental device, the surgical template and false teeth set may be configured to fit together to form a denture prosthesis that may be implanted in a patient to provide for the appearance of natural teeth.

In embodiments, the surgical template may be configured to fit over an edentulous patient's maxillary or mandibular gum tissue, and a portion of the surgical template may have the appearance of gum tissue.

In embodiments, the one or more sites may be openings each providing a channel for drilling an osteotomy into the maxillary or mandibular jaw. Further, each opening may comprise a cylindrical wall having a height which is capable of limiting the drilling depth of the osteotomy. In additional embodiments, the false teeth set comprises one or more openings configured to overlap the one or more openings of the surgical template when the template and false teeth set are fit together.

In embodiments, the surgical template and false teeth set may have male and female portions capable of interlocking. In one embodiment, the surgical template comprises a male portion configured to interlock with a female portion on one of the teeth of the false teeth set. In another embodiment, the surgical template comprises a ledge configured to fit the false teeth set.

In additional embodiments, the surgical template comprises one or more sets of drill holes configured to provide a guide for reducing jaw bone when the surgical template is positioned on the maxillary or mandibular jaw during use.

In additional embodiments, a wall of the surgical template is configured to form one or more pin holes. The one or more pin holes may be configured as projections extending outward from a wall of the surgical template. The device may further comprise pins passing though each of the pin holes. Each pin may have a handle portion extending outward from each projection and a tapered portion extending inward through each projection. In additional embodiments, the surgical template may comprise one or more flanges extending outward from the template.

Another embodiment of the invention comprises a dental device comprising a surgical template configured to fit the interior of a maxillary or mandibular jaw over an edentulous patient's maxillary or mandibular gum tissue and a false teeth set. In embodiments the surgical template and false teeth set may be configured to fit together. The surgical template may comprise one or more openings each providing a channel for drilling an osteotomy into the maxillary or mandibular jaw. Further, the false teeth set may comprise one or more openings configured to overlap the one or more openings of the surgical template when the template and false teeth set are fit together. Further, a wall of the surgical template may be configured to form one or more pin holes and the device may further comprise pins passing though each of the pin holes.

Another embodiment of the invention comprises a dental device comprising a surgical template configured to fit over the gum tissue of a partially edentulous jaw missing one or more incisors, the surgical template having one or more openings each providing a channel for drilling an osteotomy into the maxillary or mandibular jaw at the site of the missing incisors and one or more removable false teeth configured to fit the surgical template at each opening. In embodiments, the surgical template and one or more removable false teeth provide a partial or complete bridge between the left and right cuspids when implanted into a patient's jaw. In embodiments, the surgical template may be configured to partially wrap around the left and right cuspids.

An example not forming part of the claimed invention comprises a method for creating a surgical dental template, comprising performing a CT scan on a patient, transferring one or more CT scan images into treatment planning software, virtually placing implants in one or more positions on the CT scan using the treatment planning software, and creating a surgical template based on the positions on the CT scan that provide one or more sites for drilling osteotomies in the maxillary or mandibular jaw for installing the implants. In embodiments, the CT scan slices implant or anchor sites by size and diameter per zone into the jaw bone.

Another example not forming part of the claimed invention comprises a method for implanting a denture prosthesis in a patient, comprising creating a treatment planning protocol according to the treatment planning software and implanting the surgical template in the patient according to the treatment planning protocol.

In embodiments, the surgical template is configured to fit over an edentulous patient's maxillary or mandibular gum tissue. In embodiments, a portion of the surgical template has the appearance of gum tissue.

In embodiments, the one or more sites are openings each providing a channel for drilling an osteotomy into the maxillary or mandibular jaw. Each opening may comprise a cylindrical wall having a height which is capable of limiting the drilling depth of the osteotomy

In embodiments, the one or more CT scan images are in the form of DICOM files.

In embodiments, the surgical template may be manufactured from a nanoceramic composite.

In embodiments, the surgical template is manufactured through CNC milling.

In embodiments the patient is fully edentulous, and the CT scan is performed with the patient wearing a denture with radio-opaque markers. In other embodiments, the patient is partially edentulous, and the CT scan is performed with the patient wearing a radiographic guide. In other embodiments, the patient is partially edentulous, and the CT scan is performed with the patient not wearing a radiographic guide.

In examples, embodiments, the method further comprises making a set of full arch impressions from the patient's jaw; performing a CT scan on the arch impressions; and creating a surgical template based on the CT scan of the arch impressions.

In embodiments, the surgical template is configured to fit with a false teeth set to form a denture prosthesis.

In embodiments, the false teeth set comprises one or more openings configured to overlap the one or more openings of the surgical template when the template and false teeth set are fit together.

Another example not forming part of the claimed invention comprises a method for implanting or installing a denture prosthesis in a partially edentulous patient. The method may first comprise providing a surgical template and a false teeth set. The surgical template is configured to fit over the gum tissue of an edentulous jaw of a patient and has one or more sites for drilling osteotomies, and the surgical template and false teeth set are configured to fit together to form a denture prosthesis. The surgical template is then positioned over the gum tissue of an edentulous jaw of a patient at a first position and the false teeth may be inserted and set into the surgical template. The false teeth set may be fixed into the surgical template with a composite. The patient is then instructed to bite down on the false teeth set with natural teeth of the jaw opposite the edentulous jaw. The surgical template is repositioned, and the steps are repeated until the patient confirms occlusion between the natural teeth and false teeth set. The surgical template is finally fixed over the gum tissue of the patient's edentulous jaw at a second position.

In embodiments, a wall of the surgical template is configured to form one or more pin holes, the surgical template comprising pins passing though each of the pin holes, and the pins are used to fix the surgical template over the gum tissue of the patient's edentulous jaw.

In examples, embodiments, the method further comprises drilling osteotomies into the edentulous jaw based on the drilling sites and installing implants at the osteotomies and securing the surgical template through the implants. The implants can be secured with a fastener.

In embodiments, the surgical template and false teeth can be finished or converted to provide the appearance of a dental prosthesis by removing one or more of a boundary line between the surgical template and false teeth set, one or more pin holes in the surgical template, and one or more flanges in the surgical template. The boundary line and one or more pin holes can be removed by filling with composite.

Another example not forming part of the claimed invention comprises a method for reducing jaw bone. A surgical template configured to fit the interior of a maxillary or mandibular j aw over an edentulous patient's gum tissue is installed in a patient. The surgical template has one or more sets of drill holes. Next, osteotomies may be perforated through the drill holes in the surgical template to define markings in the jaw bone forming a boundary for reducing bone. The surgical template is removed and the jaw bone is exposed though an incision to reveal the osteotomies. Finally, a surgical instrument is used to remove jaw bone based on the boundary formed by the markings.

The device of the invention when implanted and converted to a denture prosthesis provides for the appearance of natural teeth while being fixed in the jaw, providing aesthetic benefits for an edentulous or partially edentulous patient. Further, the invention may provide for shorter surgery times for implantation of the device as well as greater precision in placement compared to conventional devices. In addition, the device may be converted to a temporary or final prosthesis. The device can be used with a variety of implants for implantation into jaw bone.

Another embodiment of the invention provides a dental surgery system for implanting a prosthesis, comprising a first dental surgical guide, a second dental surgical guide, and a third dental surgical guide each configured to fit a dental surgical site of a patient. The dental surgical site may be a portion of a partially edentulous maxillary of mandibular jaw of the patient. The first dental surgical guide may be configured to align with one or more teeth of the patient at the surgical site. The second dental surgical guide may be configured to allow for drilling of osteotomies for placement of implants at the surgical site. The third dental surgical guide may be configured to provide for the appearance of natural teeth such that the third surgical guide may serve as a dental prosthesis. The first dental surgical guide, second dental surgical guide, and third dental surgical guide may each comprise one or more anchoring sites for attachment to jaw bone of the patient, wherein the anchoring sites occupy the same relative positions on the first, second, and third dental surgical guides such that the guides may be aligned and secured by a single set of osteotomies determined by the anchoring sites of the first dental surgical guide.

In some embodiments, the first dental surgical guide comprises one or more voids corresponding to the positions of the one or more teeth.

In some embodiments, the second surgical guide comprises one or more voids which provide a passage for drilling of osteotomies for placement of implants at the dental surgical site.

In some embodiments, the second surgical guide comprises one or more voids which provide a passage for drilling of osteotomies for placement of implants at the dental surgical site and additionally comprises a structure for mating directly with a false teeth set.

In some embodiments, the third surgical guide comprises one or more voids which occupy the same relative positions as the one or more voids of the second dental surgical guide.

In some embodiments, the third surgical guide comprises one or more features resembling natural teeth.

In some embodiments, the one or more anchoring sites are configured to provide a channel for passage of a fastener through each of the first, second, and third dental surgical guides.

In some embodiment, the first dental surgical guide comprises one or more bone reduction sites configured to provide for the passage of a drill bit or fastener.

In some embodiments, the one or more bone reduction sites comprise a linear array of at least three channels positioned horizontally along the first dental surgical guide.

In some embodiments, the at least three channels provide a template for at least three osteotomies which map a boundary for reducing bone in the jaw.

In some embodiments, the relative positions of the at least three channels correspond to features in a CT scan of the surgical site.

In some embodiments, a bone reduction guide comprises a ridge corresponding to features in a CT scan of the surgical site.

In some embodiments, the relative positions of the one or more voids of the first surgical guide, second, and third surgical guides correspond to features in a CT scan of the surgical site.

In some embodiments, the relative positions of the one or more anchoring sites of the first, second, and third surgical guide correspond to features in a CT scan of the surgical site.

In some embodiments, the at least one of the anchoring sites on the second surgical guide is a channel configured for alignment with and placement of the second surgical guide over a reference fastener placed at the surgical site.

In some embodiments, the channel is in communication with a bottom edge of the second surgical guide.

Another example not forming part of the claimed invention provides a method of dental surgery, comprising identifying a dental surgical site of a patient, placing a first dental surgical guide at the dental surgical site through alignment of one or more features of the first dental surgical guide with one or more teeth of the patient, drilling one or more first osteotomies at the surgical site through one or more first passages in the first guide, securing the first guide at the surgical site through a fastener placed through one or more of the first passages and through each of the first osteotomies, and drilling three or more second osteotomies at the surgical site through three or more second passages in the first guide. The dental surgical site may be a portion of a partially edentulous maxillary or mandibular jaw of the patient. The three or more second osteotomies may provide a template which maps a boundary for reducing bone at the surgical site.

In some examples, the method of dental surgery may further comprise removing the first surgical guide, removing the one or more teeth of the patient at the surgical site, excising gum tissue at the surgical site to expose jaw bone and the first osteotomies and second osteotomies, and removing jaw bone at the surgical site according to the template provided by the second osteotomies.

In some examples, the method of dental surgery may further comprise placing a fastener in jaw bone at the surgical site through one of the first osteotomies, placing a second surgical guide at the surgical site through alignment of a feature of the second surgical guide with the fastener, and securing the second surgical guide through one or more passages in the second surgical guide and through one or more of the first osteotomies. The one or more passages in the second surgical guide may be in the same relative position as the one or more first passages of the first surgical guide.

In some embodiments, the feature of the second surgical guide is a channel in communication with a bottom edge of the second surgical guide.

In some examples, the method of dental surgery may further comprise drilling a set of osteotomies through voids in the second guide and placing one or more implants through the osteotomies.

In some examples embodiments, the method of dental surgery may further comprise removing the second surgical guide, placing a third surgical guide through alignment of one or more voids in the third surgical guide with the implants, and securing the third surgical guide through one or more passages in the third surgical guide and through one or more of the first osteotomies with one or more fasteners. The one or more passages in the third surgical guide may be in the same relative position as the one or more first passages of the first surgical guide. The third surgical guide may be configured to provide for the appearance of natural teeth such that the third surgical guide may function as a dental prosthesis when worn by the patient.

### Brief Description of the Drawings

The accompanying drawings illustrate certain aspects of embodiments of the present invention, and should not be used to limit the invention. Together with the written description the drawings serve to explain certain principles of the invention.

The patent or application file contains at least one drawing executed in color. Copies of this patent or patent application publication with color drawing(s) will be provided by the Office upon request and payment of the necessary fee.
FIG. 1 is a photograph showing a prior art surgical guide half prosthesis.
FIG. 2A is a photograph showing a prior art tooth-supported poly(methyl methacrylate) (PMMA) Milled Split Surgical Guide.
FIG. 2B is a photograph showing a prior art tooth- and pin-supported PMMA Milled Split Surgical Guide.
FIG. 3A is a photograph showing a front view of a fully assembled combination surgical template and dental prosthesis device according to an embodiment of the invention.
FIG. 3B is a photograph showing a front, oblique view of a fully assembled combination surgical template and dental prosthesis device according to an embodiment of the invention.
FIG. 3C is a photograph showing a top view of a maxillary portion of a combination surgical template and dental prosthesis device according to an embodiment of the invention.
FIG. 4 is a photograph showing a mandibular portion of a combination surgical guide dental prosthesis device according to an embodiment of the invention.
FIG. 5 is a photograph showing a surgical template converted to dental prosthesis with pins, pin holes, and flanges removed according to an embodiment of the invention.
FIG. 6A is a photograph showing a front view of an embodiment of the surgical template converted to a temporary dental prosthesis with pins, pin holes, and flanges removed according to an embodiment of the invention.
FIG. 6B is a photograph showing a bottom view of an embodiment of the surgical template converted to a final dental prosthesis showing towers from multi-unit abutments.
FIG. 7 is a photograph showing a two-part design of a combination surgical template and dental prosthesis device according to an embodiment of the invention.
FIG. 8A is a photograph showing a front, oblique view of a drilling sequence of a combination surgical template and dental prosthesis device according to an embodiment of the invention.
FIG. 8B is a photograph showing a top view of a drilling sequence of a combination surgical template and dental prosthesis device according to an embodiment of the invention.
FIG. 8C is a photograph showing a front view of a drilling sequence of a combination surgical template and dental prosthesis device according to an embodiment of the invention.
FIGS. 9A-9D are photographs showing a chair-side adaptation workflow according to an embodiment of the invention.
FIG. 10A is a photograph showing bars milled for final clone prosthesis according to an embodiment of the invention.
FIG. 10B is a photograph showing bars embedded in a nanoceramic prosthesis according to an embodiment of the invention.
FIG. 11A is a photograph showing a front view of a patient implanted with a converted surgical guide and dental prosthesis device according to an embodiment of the invention.
FIG. 11B is a photograph showing a front view of a patient implanted with a converted surgical template and dental prosthesis device according to an embodiment of the invention with teeth in occlusion.
FIG. 11C is a photograph showing a side view of a patient implanted with a converted surgical template and dental prosthesis device according to an embodiment of the invention with teeth in occlusion.
FIG. 12 is a photograph showing a combination surgical template and dental prosthesis device with bone reduction guide according to an embodiment of the invention.
FIG. 13 is a photograph showing a partial surgical template and dental prosthesis device according to an embodiment of the invention.
FIG. 14 is a photograph showing a front view of a drilling sequence of a partial surgical template and dental prosthesis device according to an embodiment of the invention.
FIG. 15A-15B are photographs before (FIG. 15A) and after (FIG. 15B) of a patient implanted with a partial surgical template and dental prosthesis device according to an embodiment of the invention.
FIGS. 16-17 are computer-aided design (CAD) drawings showing the combination surgical template and dental prosthesis device of the invention is fully compatible with the BIOHORIZONS® Guided Surgery Kit according to an embodiment of the invention.
FIGS. 18A-F are CAD drawings showing a series of steps for using a surgical template of the invention with the TEETHXPRESS® immediate load solution according to an embodiment of the invention.
FIG. 19A is a CAD drawing showing an example of BIOHORIZONS® implants according to an embodiment of the invention.
FIG. 19B is a CAD drawing showing an example of BIOHORIZONS® multiunit abutments according to an embodiment of the invention.
FIG. 20 is a screenshot showing an example of a scanning protocol verification checklist according to an embodiment of the invention.
FIG. 21 is a CAD drawing showing the placement of implants according to an embodiment of the invention.
FIG. 22 is a screenshot of a Treatment Planning Report Overview supplied for case analysis according to an embodiment of the invention.
FIG. 23 is a screenshot of a Planning Report for an implant site for critical evaluation according to an embodiment of the invention.
FIG. 24 is a screenshot showing guided surgery information for a patient according to an embodiment of the invention.
FIG. 25 is a screenshot showing a treatment planning approval form according to an embodiment of the invention.
FIG. 26A is a photograph of a guided surgery kit according to an embodiment of the invention.
FIG. 26B is a photograph showing fast healing using a guided surgery method not forming part of the claimed invention;
FIG. 27 is a flow chart of a decision tree to support the appropriate scanning protocol according to an embodiment of the invention.
FIGS. 28A-28D are screenshots showing a Single Scan Protocol for Teeth-supported Surgical Guide according to an embodiment of the invention.
FIGS. 29A-29E are screenshots showing a Dual Scan Protocol for Teeth-supported Surgical Guide according to an embodiment of the invention.
FIGS. 30A-30F are screenshots showing a Dual Scan Protocol for Gum-Supported Surgical Guide according to an embodiment of the invention.
FIGS. 31A-31D are screenshots showing file transfer instructions according to an embodiment of the invention.
FIG. 32 is a flowchart illustrating a method for creating a surgical dental template not forming part of the claimed invention.
FIG. 33 is a flowchart illustrating a method for implanting a denture prosthesis in a partially edentulous patient not forming part of the claimed invention.
FIG. 34 is a flowchart illustrating a method for reducing jaw bone in a patient not forming part of the claimed invention.
FIG. 35 is a CAD drawing showing a tooth-borne surgical guide (or first guide) according to an embodiment of the invention.
FIG. 36 is a CAD drawing showing a bone model of a patient showing based on placement of the guide of FIG. 35 according to an embodiment of the invention.
FIG. 37 is a CAD drawing showing an osteotomy guide (or second guide) according to an embodiment of the invention.
FIG. 38 is a CAD drawing showing a prosthetic guide (or third guide) according to an embodiment of the invention.
FIG. 39 is a photograph showing the mouth of a partially edentulous patient that represents the subject case of subsequent figures.
FIG. 40A-40C are photographs showing a left (FIG. 40A), front (FIG. 40B), and right (FIG. 40C) perspective view of the teeth of the subject case.
FIG. 41A is a photograph showing a bottom perspective view of implants of the maxillary jaw of the subject case.
FIG. 41B is a photograph showing a top perspective view of the existing teeth of the mandibular jaw of the subject case.
FIGS. 42A-42C are CAD drawings showing a left (FIG. 42A), front (FIG. 42B), and right (FIG. 42C) perspective views of a CT treatment plan for the subject case.
FIGS. 43A and 43B are CAD drawings showing an occlusal view of a CT treatment plan for the subject case.
FIGS. 44A are photographs showing a top view of the first guide or tooth-bome surgical guide according to an embodiment of the invention.
FIG. 44B is a photograph showing a top view of the second guide or osteotomy guide according to an embodiment of the invention.
FIG. 44C is a photograph showing a top view of the third guide or prosthetic guide according to an embodiment of the invention.
FIGS. 45A-45C are photographs showing a left (FIG. 45A), front (FIG. 45B), and right (FIG. 45C) perspective view of the first guide or tooth-bome surgical guide according to an embodiment of the invention.
FIGS. 46A and 46B are photographs showing placement of the tooth-bome surgical guide at a surgical site of the subject.
FIGS. 47A and 47B are photograph showing show fit verification of the tooth-supported surgical guide.
FIG. 48A is a photograph shows the drilling of osteotomies for the anchoring sites, while FIG. 48B is a photograph showing fixation of the guide in the jaw of the subject through the placement of fixation (or anchoring) pins.
FIGS. 49A-49C are photographs showing drilling of the channels of the bone reduction sites provided by the first surgical guide.
FIGS. 50A and 50B are before and after photographs showing extraction of remaining teeth exposure of the jaw bone and drilling sites through excision of gum tissue.
FIG. 51A is a photograph showing the guide pins reinserted into the jaw bone for visual purposes.
FIGS. 51B and 51C are photographs showing identification of the bone reduction sites and bone removal around the arch through a "connect the dots" method.
FIGS. 52A and 52B are photographs showing that bone removal is complete.
FIGS. 53A-53C are photographs showing left (FIG. 53A), front (FIG. 53B), and right (FIG. 53C) perspective views, respectively, of an implant osteotomy guide according to an embodiment of the invention.
FIG. 54 is a photograph showing one of the insertion pins is placed in jaw bone to provide a reference point for the implant osteotomy guide.
FIGS. 55A and 55B are photographs showing alignment of the insertion pin with the channel of the osteotomy guide during placement of the guide, so that the guide is properly orientated at the surgical site.
FIGS. 56A-56C are photographs showing the remaining anchoring sites are pinned to secure the implant osteotomy guide in the jaw at the surgical site.
FIG. 57 is a photograph showing the osteotomy guide installed at the surgical site.
FIGS. 58A-C are photographs showing drilling of osteotomies through voids in the osteotomy guide.
FIG. 59A is a photograph showing a tool for performing osteotomies.
FIGS. 59B and 59C are photographs showing additional drilling of osteotomies through voids in the osteotomy guide.
FIG. 60A is a photograph showing a drill for installing implants.
FIGS. 60B and 60C are photographs showing installation of implants into the drilled osteotomies.
FIGS. 61A-61C are photographs showing implants being placed with a driver.
FIG. 62 is a photograph showing the implants fully seated.
FIGS. 63A-63C are photographs showing insertion of multi-unit abutments.
FIGS. 64A and 64B are photographs showing the abutments installed.
FIGS. 65A and 65B are photographs showing installation of temp copings on top of the abutments.
FIGS. 66A-66C are photographs showing a left (FIG. 66A), front (FIG. 66B), and right (FIG. 66C) perspective view of the third guide (prosthetic guide).
FIGS. 67A-67B are photographs showing placement of the third guide in the lower jaw.
FIGS. 68A and 68B are photographs showing application of a light-cured composite or acrylic (FIG. 68A) to voids in the third guide and curing with light (FIG. 68B)
FIGS. 69A-69C are photographs showing remaining voids in the prosthesis are filled with light-cured composites or acrylics outside the mouth.
FIGS. 70A-70C are photographs showing different perspective views of the final prosthesis with insertion pins and pin holes trimmed, flanges reduced, and convex intaglio.
FIGS. 71A and 71B are photographs showing top and bottom views of the prosthesis guide.
FIG. 72 is a photograph showing the final mandibular prosthesis guide in the mouth.
FIGS. 73A-73C are photographs showing occlusion of the top and bottom prostheses.
FIG. 74 is a photograph showing an X-ray image of the patient, which shows placement of the implants.
FIG. 75 is a CAD drawing of the superstructure/bar.
FIG. 76 is a CAD drawing of the mandibular false teeth with a mating groove for the superstructure/bar.
FIG. 77 is a CAD drawing of the superstructure mated with mandibular false teeth.
FIG. 78 is a CAD drawing of the bone reduction jig.
FIG. 79 is a photograph showing the superstructure with teeth-shaped protrusions.

### Modes for Carrying Out the Invention

Reference will now be made in detail to various exemplary embodiments of the invention. It is to be understood that the following discussion of exemplary embodiments is not intended as a limitation on the invention. Rather, the following discussion is provided to give the reader a more detailed understanding of certain aspects and features of the invention.

In one embodiment, the present invention is a two-part denture prosthesis or device comprising a surgical template or guide and removable false teeth set for both the mandibular and maxillary jaw. The surgical template and false teeth set may be configured to fit together in an interlocking configuration. The surgical guide may exist as two components that are each configured to fit the top (maxilla) or bottom (mandible) portion of a patient's jaw when placed inside the mouth. The surgical guide may have one or more holes that may serve as drilling sites. The holes may be positioned so that osteotomies may be drilled downward in the mandible or upward in the maxilla. The holes in the guide can comprise a cylindrical wall (also known herein as a depth stop) that is raised or lowered to accommodate a target depth of the osteotomy. The surgical guide may have one or more pin holes for securing or clamping the guide to the jaw during surgery. During surgical placement of the guide, pins can be set through the pin holes in the guide through the bone to secure the guide in place until it is permanently fixed with the abutments or implants. In embodiments, the pin holes are located laterally on the guide and spaced apart to secure the sides and central portion of the guide.

In embodiments, the surgical guide has a removable false teeth set configured to interlock with the surgical guide. The removable teeth may exist as two additional components (e.g., one for the maxillary jaw and one for the mandibular jaw) each configured to fit the top maxillary portion or bottom mandibular portion of the surgical guide. The removable false teeth set allows the oral surgical guide to be simultaneously used as a prosthesis. In addition, the removable false teeth are useful in helping to confirm the fit of the surgical guide through occlusion. The removable teeth may fit on the surgical guide above or below the drill holes and may have access holes that complementarily fit the drill holes. Ultimately, the surgical guide and teeth component are secured as one piece to the implant(s) placed in the jaw though a fastener such as screws.

The surgical guide may be manufactured to fit the individual patient's mouth based on a CT scan of the maxilla and mandible. The surgical guide may be manufactured from a nanoceramic composite or other suitable material through CNC milling or 3D printing.

In another example, the disclosure relates to a method for performing oral surgery. The method comprises placing a surgical guide of the invention in a patient's mouth, setting one or more pins through the holes of the guide to secure the guide to the jaw, placing a set of teeth on the guide, instructing the patient to bite down on the teeth to confirm the fit of the guide through occlusion, if necessary, releasing the guide with the pins and readjusting the fit of the guide based on the occlusive bite, and securing the guide with the pins, drilling osteotomies through the holes, and securing the guide to the jaw through implants or abutments placed in the osteotomies. The methods may further comprise using a pre-surgical bite registration index based on the patient's bite. The methods may further comprise cutting off insertion pin holes and flanges on the guide and filling the border between the surgical guide and false teeth set with composite to provide for a natural look.

In another example, the disclosure relates to a method of placing a dental prosthesis in the mouth of a patient. The method comprises placing a surgical guide in the mouth of a patient, performing guided surgery based on drill holes in the surgical guide, inserting abutments through the surgical guide, retrieving the surgical guide and reducing excess material, inserting a complementary false teeth set into the surgical guide, drilling a guided screw channel into the surgical guide, and finishing the surgical guide.

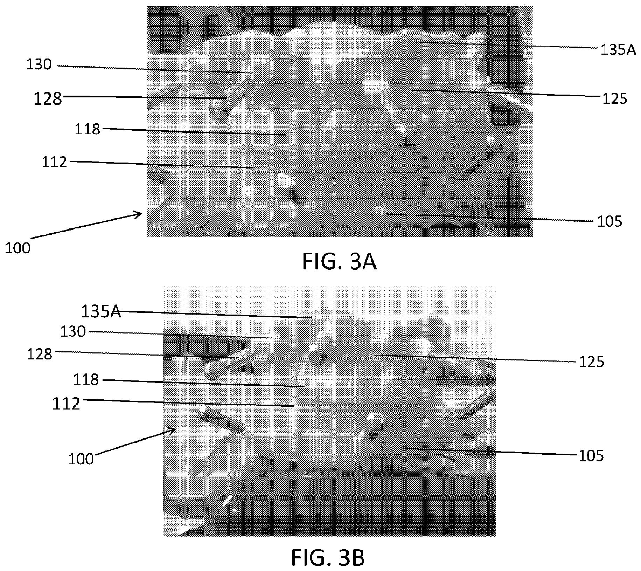

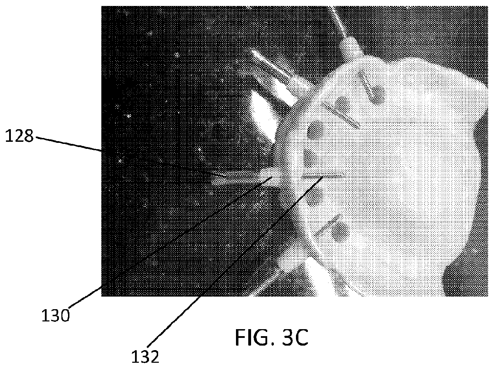

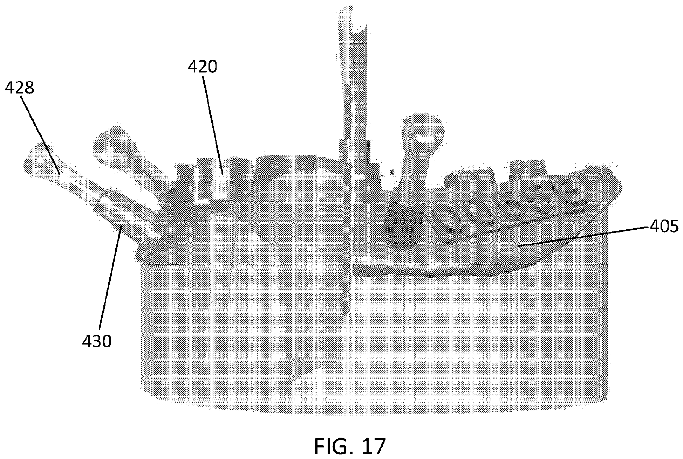

Various embodiments of a device of the invention are shown in FIGS. 3-18F. FIGS. 3A-3B show a manufactured/milled denture prosthesis 100 according to an embodiment of the invention. The denture prosthesis includes mandibular guide 105, mandibular false teeth set 112, maxillary guide 125, and maxillary false teeth set 118. Guides 105 and 125 include insertion pin holes 130 and insertion pins 128 as well as flanges 135A extending above the guides. Guides 105, 125 may have portions with the color, shape, and appearance of gum tissue. The insertion pins 128 may be used to pin the guide in position into the jaw bone based on results of the CT scan and holds the guide in place like a vise. The false teeth sets 112 and 118 are used as a reference for orientating the guides 105 and 125 into the jaw during placement. The guide is first preliminarily placed in the mouth based on the results of the CT scan, the teeth are placed in the guide, and the patient's mouth is closed to provide occlusion of the false teeth set with the natural teeth set on the opposite jaw. This method is also called canine guidance, in which occlusal contacts of the cuspids cause contacts of posterior teeth to separate in excursive mandibular movements. Occlusion confirms that the guide is in proper position. The false teeth sets 112 and 125 may be glued to the guides 105 and 125 during final preparation of the dental prosthesis. FIG. 3C shows that insertion pins 128 have a tapered portion 132 projecting inward through and beyond insertion holes 130 for insertion into the jaw.

As will be shown below, the false teeth sets 112, 118 and guides 105, 125 may include features that allow corresponding components to fit into place. These may include at least one male component on the guides 105, 125 and at least one female component on the teeth or vice versa. In one embodiment, the guides 105, 125 have a male component having an appearance of a molar tooth structure that interlocks with a molar tooth structure of the corresponding false teeth sets 112, 118. In other embodiments, the guides 105, 125 have a ledge or gulley that is configured to accept a protruding portion of the corresponding false teeth sets 112, 118. Once they are fit together, false teeth sets 112, 118 and guides 105, 125 may be permanently fixed together through an adhesive such as a composite.

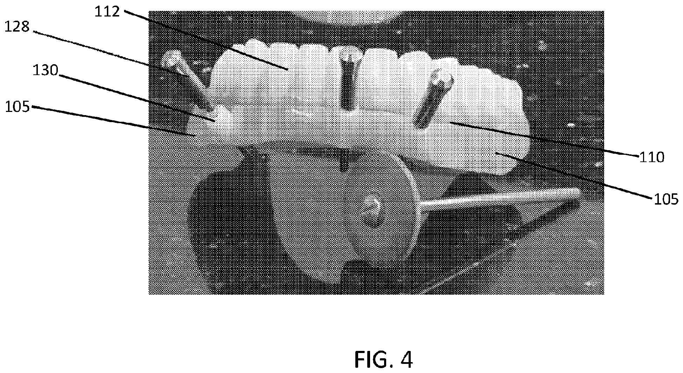

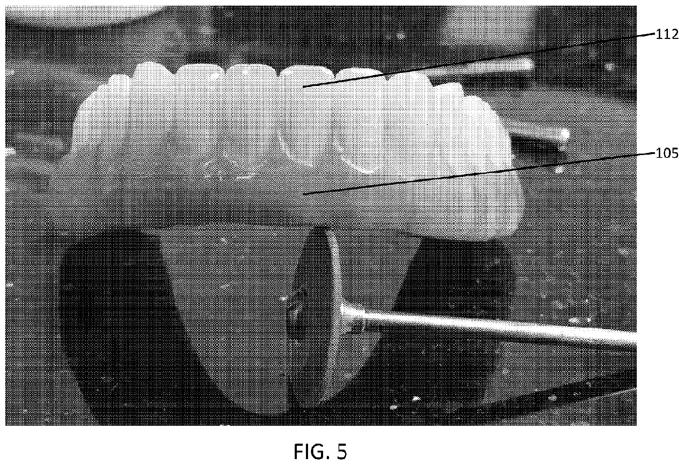

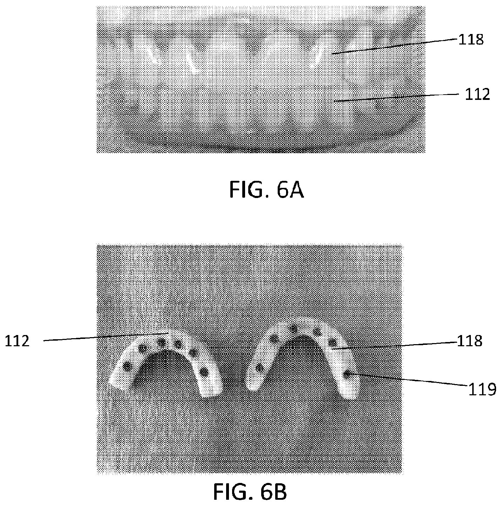

FIG. 4 includes a surgical guide 105 and false teeth set 112 which includes line 110 at point of insertion of false teeth set 112 into surgical guide 105. Since the surgical guide 105 and false teeth set 112 will serve as a prosthesis, unnatural features such as insertion pin holes 130 and line 110 must be removed from the device during a conversion process. FIGS. 5 and 6A shows a surgical guide 105 and false teeth set 112 with line between them removed by filling with composite and insertion pin holes removed. The surgical guide 105 and false teeth set 112 shown in FIG. 5 is processed according to how it would appear in a patient. FIG. 6A shows the maxillary 118 and mandibular 112 false teeth sets together in occlusion. FIG. 6B is a photograph showing a bottom view of an embodiment of the surgical template converted to a final dental prosthesis showing towers 119 from multi-unit abutments.

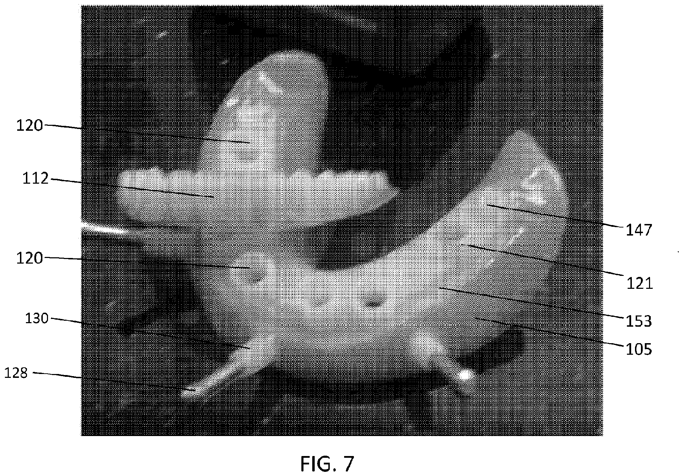

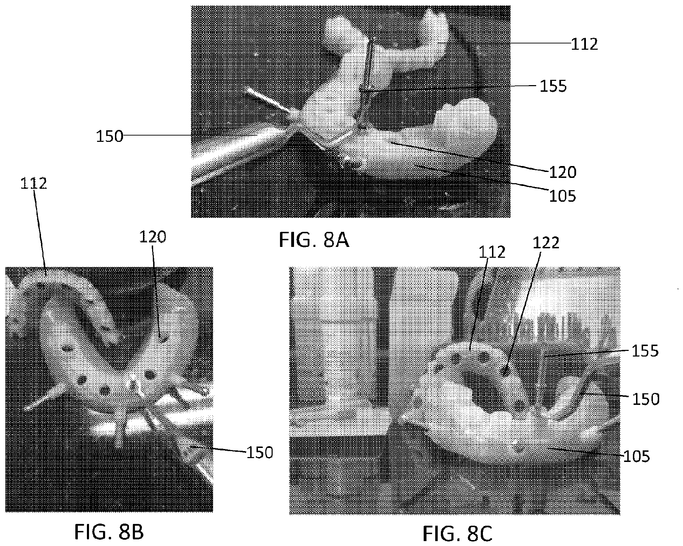

Turning now to FIG. 7, shown is a surgical guide 105 with separated false teeth set 112, which reveals drilling sites 120 on the surgical guide 105. The drilling sites 120 comprise a cylindrical hole which may be provided at different depths to help guide the depth of the drill hole. For example, a cylindrical hole may include a wall 121 at a specified height to prevent drilling too deep. These cylindrical hole configurations are also known as depth stops. As shown in FIGS. 8A-8C, the drilling sites 120 may be accessible through guided surgical drilling equipment 150, 155, which may drill osteotomies in the jaw for fixing the surgical guide 105 and false teeth set 112 into the jaw. Also shown in FIG. 8C are complementary holes 122 in the false teeth set which align with the drilling sites 120 which serve as screw access holes.

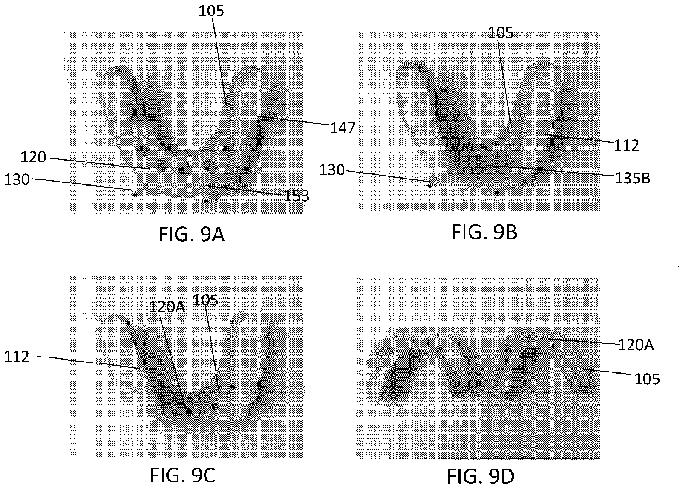

Now referring to FIGS. 9A-9D, shown are a surgical guide and dental prosthesis device of the invention at different stages of assembly. FIG. 9A shows a mandibular surgical guide 105 with the removable false teeth set 112 removed, while FIG. 9B shows the mandibular surgical guide 105 with the removable false teeth set 112 attached. Also shown in FIGS. 7 and 9A are interlocking structure 147 and horseshoe-shaped ledge 153 for connecting false teeth set 112 to mandibular surgical guide 105. Such features may be used to fit the two components in place. FIGS. 9C and 9D show the surgical guide 105 with excess material removed, including the interior drilling sites 120A, flange 135B, and insertion pin holes 130 shown in FIGS. 9A and 9B. In embodiments, the excessive material reduction is a component of a Chair-Side Adaptation Workflow, which includes the following steps:
1. Guided surgery
2. Abutment (not shown) insertion and bonding, through surgical guide still installed
3. Surgical guide retrieval and excess material reduction
4. Surgical guide parts assembly and bonding
5. Guided screw channel drilling
6. Finishing

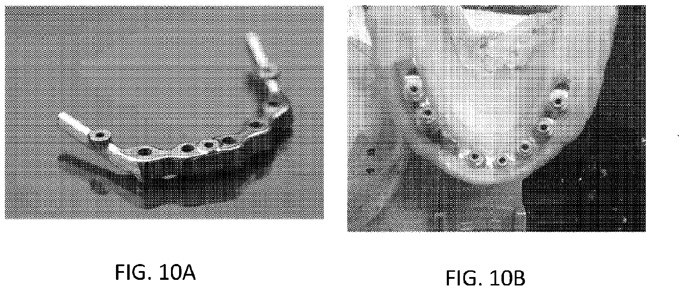

In embodiments, the device of the invention may be used as a temporary prosthesis until a more permanent prosthesis is put into place. FIGS. 10A and 10B show the manufacture of a more permanent prosthesis based on the device. FIG. 10A is a photograph showing bars milled for final clone prosthesis according to an embodiment of the invention. FIG. 10B is a photograph showing bars embedded within a nanoceramic prosthesis according to an embodiment of the invention. The more permanent prosthesis may be implanted in the jaw through the implants installed through the surgical guide.

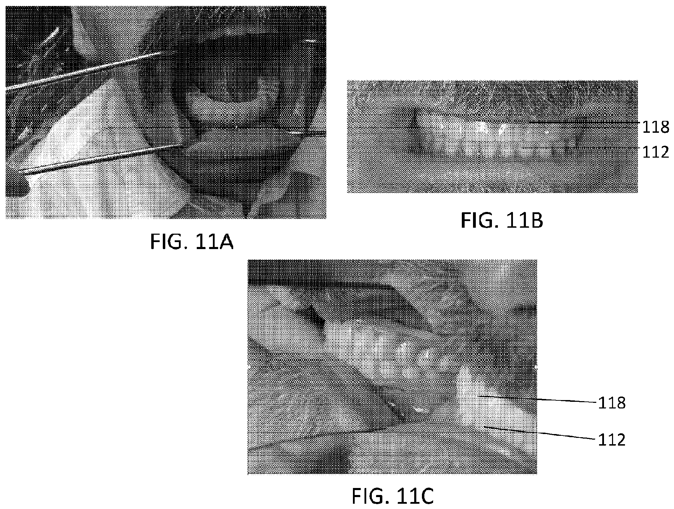

FIGS. 11A-11C show a patient implanted with a surgical guide converted to a dental prosthesis according to an embodiment of the invention. As shown in FIGS. 11B and 11C, the maxillary 118 and mandibular 112 false teeth sets are positioned so that they are in perfect occlusion with canine guidance.

In embodiments, the surgical guide may include one or more retaining pins and drill holes located on the denture which serve as a surgical guide for bone reduction. The bone reduction guide assists the user to reduce bone to the necessary thickness in order for the denture to be placed at the right height, and may be determined from a CT scan of the patient's jaw. The retaining pins and drill holes allow for a boundary on the jaw to be marked when the surgical guide is placed in the jaw as the jaw may be marked by drilling though the drill holes. When the guide is placed the jaw may be exposed through an open flap incision, allowing the oral surgeon to see the holes. The jaw may be reduced by sawing down the jaw until the holes are no longer visible.

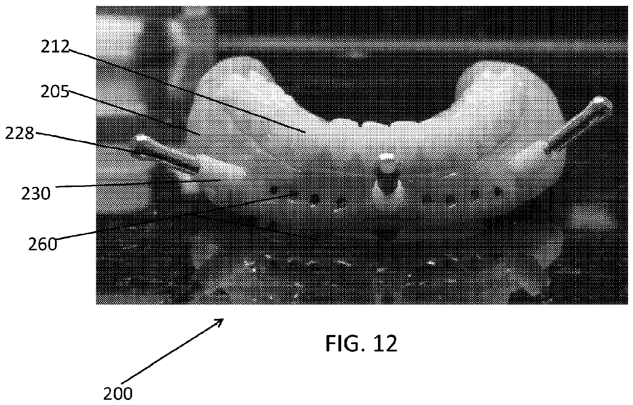

Now referring to FIG. 12, a partial denture prosthesis 200 comprising a surgical guide 205 with bone reduction guide according to an embodiment of the invention is shown. FIG. 12 shows three retaining pins 228 located in insertion pin holes 230 and two sets of four bore holes 260 located on surgical guide 205, which like previous embodiments has corresponding false teeth set 212. The bore holes 260 are drilled into the surgical guide 205, which provides a bone reduction guide for the device. It acts as a part of the same medical device. The bone reduction guide assists the user to reduce bone to the necessary thickness which may be 15-16 millimeters thick in order for the denture to be placed at the right height. The ridge width acts as a guide to remove the bone. The user will push through the tissue and make holes in the bone. Pins inserted into the tissue will be cut. The gum tissue will be opened up and the bone will be exposed. When this happens there will be holes in the bone. The bone will be grinded until the holes disappear at which point the bone will be leveled down along the peak of the mountain peak which is the front or anterior end of the jaw. The surgeon will remove bone along the holes. There will be depressions in the bone and the bone will be ground down until there is a flat plane. The osteotomy is CT driven and will use a denture wherein it will be filled with resin. A denture will be used to get inside of bone. It acts as a surgical guide, prosthesis, and denture guide.

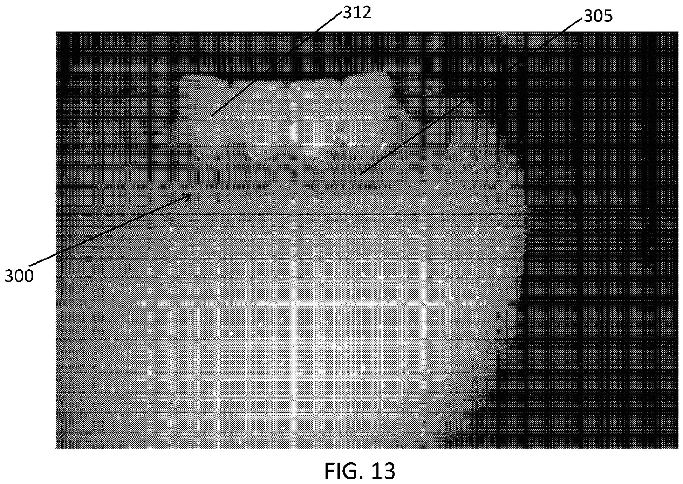

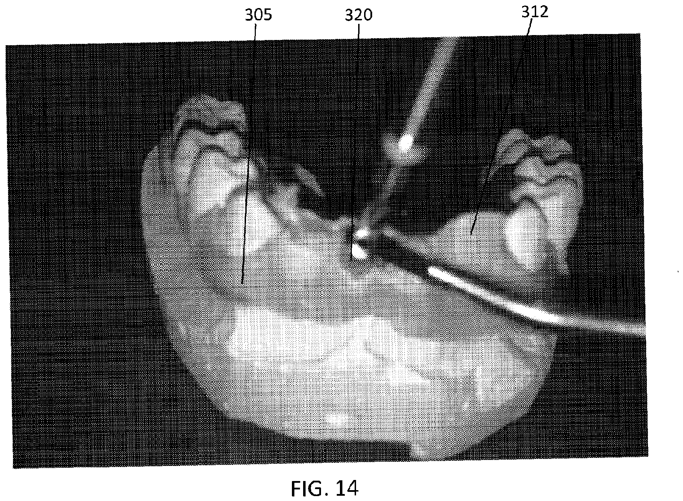

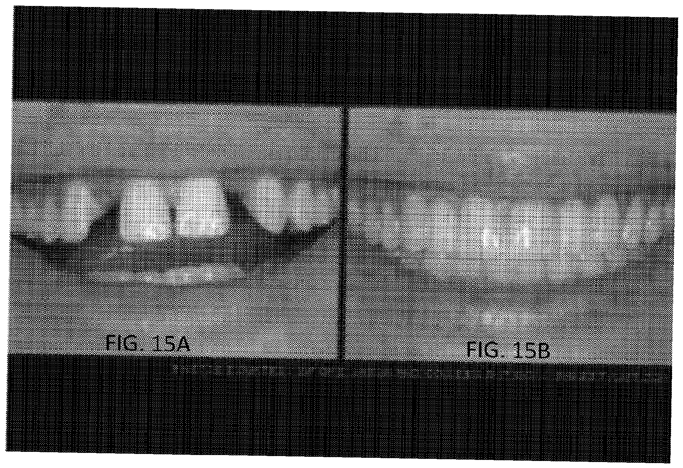

FIGS. 13 and 14 show a partial denture prosthesis embodiment 300 according to the invention. Partial denture 300 includes surgical guide 305 and removable false teeth set 312. When teeth 312 are removed they reveal drill holes 320 which are accessed by drilling equipment. FIGS. 15A and 15B show before after photographs of a patient surgically implanted with the partial denture 300. In embodiments, the partial denture comprises a false teeth set having anywhere from 2 to 8 teeth.

As shown in FIGS. 13 and 14, a surgical guide or template 305 is configured to fit over the gum tissue of a partially edentulous jaw missing one or more incisors. The surgical template 305 has one or more openings 320 each providing a channel for drilling an osteotomy into the maxillary or mandibular jaw at the site of the missing incisors. Included are one or more removable false teeth 312 configured to fit the surgical template at each opening 320. As shown in FIGS. 14, 15A, and 15B, the surgical template and one or more removable false teeth provide a partial or complete bridge between the left and right cuspids when implanted into a patient's jaw. In embodiments, the surgical template is configured to partially wrap around the left and right cuspids to secure it to the jaw.

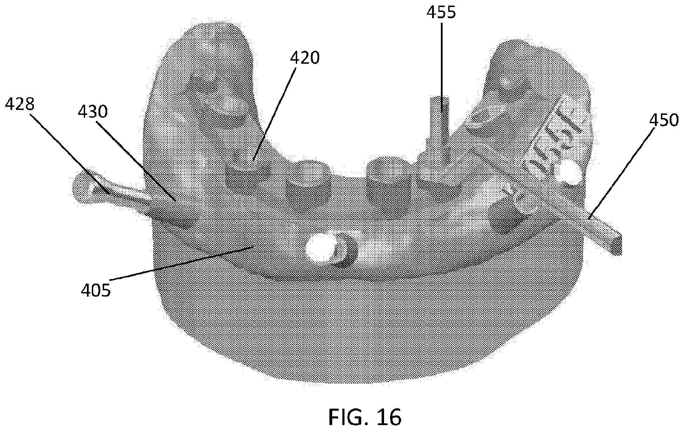

FIGS. 16-17 are CAD drawings showing the surgical guide of the invention is fully compatible with the BIOHORIZONS® Guided Surgery Kit according to an embodiment of the invention. FIGS. 16-17 show guide 405 with insertion pins 428 and insertion pin holes 430, and drilling holes 420 which are accessible by drilling equipment 450, 455. Features of the surgical guide include anchoring using the CGS-FP Fixation pins, secure tool assembly using the embedded pilot drill sleeves or yellow, green and blue Master Cylinders, site preparation using the GGS-YTP/GTP/BTP Tissue Punches, guided osteotomy using BIOHORIZONS® Drill Guides and Drills, all displayed during design to prevent interference with adjacent geometry, guided insertion using the BIOHORIZONS® Implant Drivers and Depth Stops. The four depth stop configurations can be considered during treatment planning.

The following provides specifications of a Demo Case:
Master cylinder = Green
Drill guide = Green, 4.1mm internal diameter (final)
Implant = 4.6mm x 10.5mm Tapered
Internal Implant = Drill 21 mm length, 4.1mm diameter (final)
Implant driver and depth position = Tapered Internal 4.6, SP2

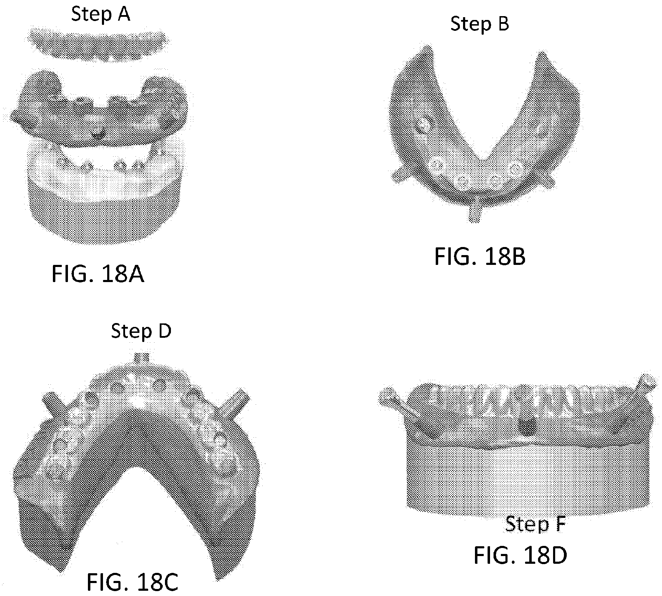

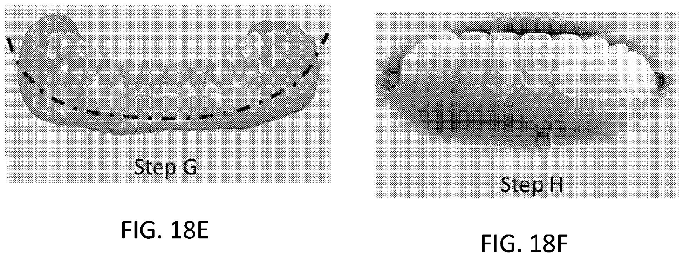

FIGS. 18A-F are CAD drawings showing a series of steps for using the surgical guide of the invention with the TEETHXPRESS® immediate load solution according to an embodiment of the invention. Such steps are explained in Table 1 below.

**Table 1: Compatibility with TEETHXPRESS®**

| **Steps** | **TEETHXPRESS®** | **TEETHXPRESS® + Surgical Guide** | **Corresponding Figures** |
|---|---|---|---|
| A. Starting conditions: | •Implants with adequate primary stability and proper abutments installed | •Implants with adequate primary stability and proper abutments install ed | FIG. 18A |
| | • Denture | •Surgical Guide | |
| B. Initial modifications: | •Preparation of a denture hole drilling template | •Prefabricated holes aligned according to the treatment plan (with the implants on the baseplate, and with the angled multiunit abutment on the halfprosthesis) | FIG. 18B |
| | •Drilling and adjustment oftheholesto accommodate theabutments*(Steps 1 to 3)* | •Slight adjustments of the holes to accommodate the abutments and copings | |
| C. Occlusion and passive fit verification: | •Using the pre-surgical bite registration index *(Step 4)* | •Using the halfprosthesis, and a pre-surgical bite registration index for cases with challenging prosthetic geometry | |
| D. Coping reduction and installation: | •Coping installation, marking, trimming, smoothing, polishing and installation with rubber dam *(Steps 5 to 11)* | •Trim any protruding drill guide supporting cylinders from the baseplate | FIG. 18C |
| | | •Coping installation, marking, trimming, smoothing, polishing and installation with rubber dam | |
| E. Occlusion and intercuspation verification: | •Using the pre-surgical bite registration index *(Step 12)* | •Using the halfprosthesis, and a pre-surgical bite registration index for cases with challenging prosthetic geometry | |
| F: Coping pick-up: | •Using self curing acrylic | •Using chairside flowable, lightcurable composite | FIG. 18D |
| | •Using the pre-surgical bite registration index for stability during acrylic curing *(Steps 13 to 17)* | •Using the Surgical Guide anchor pins for accuracy and stability during composite curing | |
| G. Prosthesis adaptation: | •Retrieve the prosthesis | •Retrieve the prosthesis | FIG. 18E |
| | •Fill-in any void around the abutments/copings with acrylic | •Trim the lot number and guide anchors | |
| | •Shorten flanges and any excessive posterior cantilever | •Assemble the halfprosthesis to the base-plate with composite | |
| | •Creation of a 2mm incision from the mucosa *(Steps 18 to 20)* | •Fill-in any void with composite | |
| | | •Shorten flanges and any excessive posterior cantilever | |
| | | •Creation of a 2mm incision from the mucosa | |
| H. Occlusion verification, polishing and delivery of the prosthesis: | •Using the pre-surgical bite registration index | •Using the halfprosthesis, and a pre-surgical bite registration index for cases with challenging prosthetic geometry | FIG. 18F |
| | •Cotton filling and sealing of the screw access holes | | |
| | •Acquisition of a post-delivery panoramic x-ray to validate the seating of the prosthesis *(Steps 21 to 24)* | •Cotton filling and sealing of the screw access holes | |
| | | •Acquisition of a post-delivery panoramic x-ray to validate the seating of the prosthesis. | |

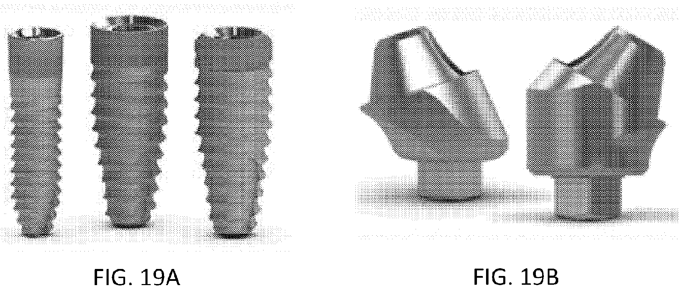

FIG. 19A is a CAD drawing showing an example of BIOHORIZONS® implants according to an embodiment of the invention. FIG. 19B is a CAD drawing showing an example of BIOHORIZONS® multiunit abutments according to an embodiment of the invention. These are examples of implants and abutments that can be used to secure the surgical template of the invention to the jaw. However, the device of the invention is compatible with a variety of implants such that the surgical template may be considered a universal guide.

In another example, the disclosure relates to
a method of creating a two-part dental prosthesis comprising a surgical guide and false teeth set. The method comprises performing a CT scan on a patient, transferring the scan (such as in the form of a Digital Imaging and Communications in Medicine (DICOM) file) into treatment planning software. The CT scan slices implant or anchor sites by size and diameter per zone into the jaw bone. From the CT scan, a surgical guide is created based on one or more anchor or implant sites to create a surgical template. The surgical guide may be created by a trained biomedical engineer or oral surgeon who virtually places the implants in position on the CT scan, taking into account the bone quality and quantity and the presence of nerves. The surgical guide is then milled based on the template that provides one or more holes that may serve as drilling sites to place the implants which secure the guide into the jawbone. The drilling sites are created so that the implants are placed at consistent angles to provide for an even pressure distribution on the surgical guide when it is secured in the jaw. A treatment protocol may also be produced from the treatment planning software which instructs a dental surgeon how to place the surgical guide, including the drilling depth. Based on the CT scan, sites in the jaw bone may be identified that may be used to secure one or more implants. The implants may provide a means for securing the device in place with a fastener such as a screw. The device may be attached to the jaw through the implants according to the implant manufacturer's instructions.

In embodiments, the CT scan may be performed in a variety of ways, depending on whether the jaw is fully or partially edentulous and whether metal restorations or amalgams are present. For a partially edentulous jaw, if there is good teeth support, and no metal restorations or amalgams, then the CT scan may be performed without a radiographic guide. If there is good teeth support and amalgams or metal restorations are present, then the CT scan may be performed with a radiographic guide. For a partially edentulous jaw without good teeth support or for a fully edentulous jaw, the CT scan may be performed with a gum-supported surgical guide (denture with markers).

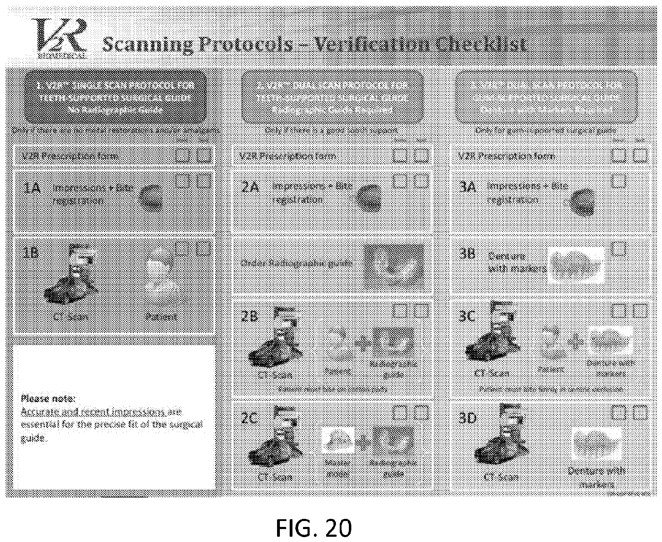

FIG. 20 is a screenshot showing an example of a scanning protocol verification checklist according to an embodiment of the invention, illustrating different scanning protocols that can be used to create the surgical guides. The checklist includes the following separate protocols: Single-Scan Protocol for Teeth-Supported Surgical Guide, Dual Scan Protocol for Teeth-Supported Surgical Guide, and Dual Scan Protocol for Gum-Supported Surgical Guide. The Single Scan Protocol can be used if there are no metal restorations or amalgams. The Dual Scan Protocol for Teeth-Supported Surgical Guide can be used only if there is good tooth support, and the Dual Scan Protocol for Gum-Supported Surgical Guide can be used for the gum-supported surgical guide. Further, the Single Scan protocol can be used without any radiographic guide, the Dual Scan Protocol for Teeth-Supported Surgical Guide is used with a radiographic guide, and the Dual Scan Protocol for Gum-Supported Surgical Guide is used with a denture with markers.

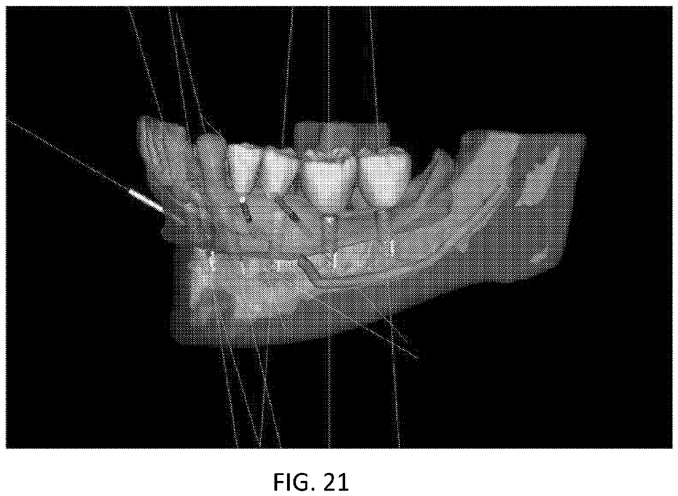

FIG. 21 is a CAD drawing showing the placement of implants according to an embodiment of the invention.

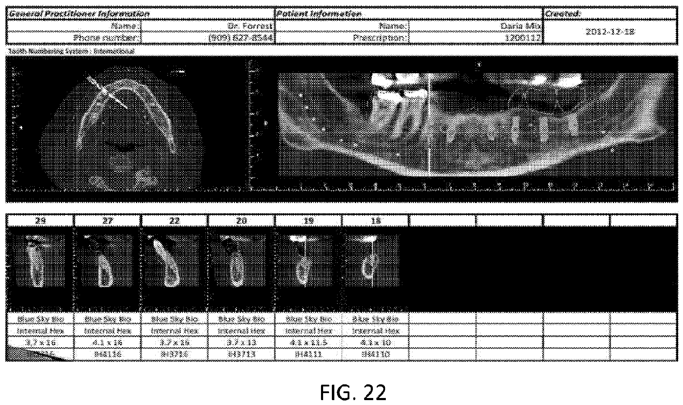

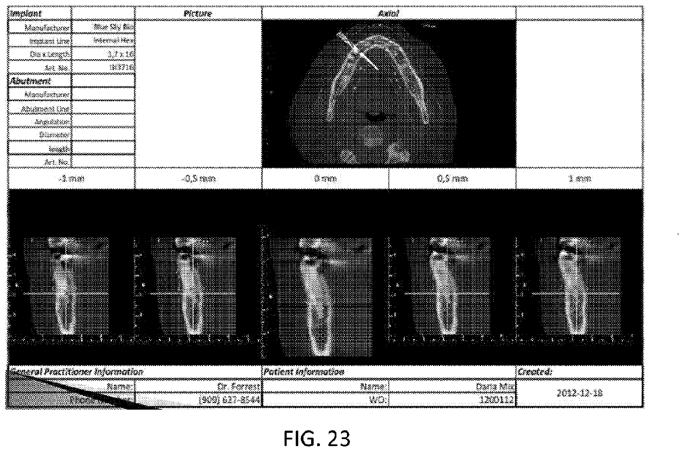

FIG. 22 is a screenshot of a Treatment Planning Report Overview supplied for case analysis according to an embodiment of the invention. The Treatment Planning Report Overview includes results of the CT scan of the whole jaw (top) as well as slices of the individual anchor or implant sites (bottom). The CT scan slices zones for each of the implant sites. If this step if not performed, the implants may be placed at divergent angles, and too much pressure may be placed on the implants and they may ultimately fail. The guide must be oriented so that the implants are not coming down through the teeth or the gums. They should be parallel and in best position in concert with the bone such that everything lines up. Based on the results of the Treatment Planning Report Overview, a Planning Report for each implant site is provided, an example of which is shown in FIG. 23.

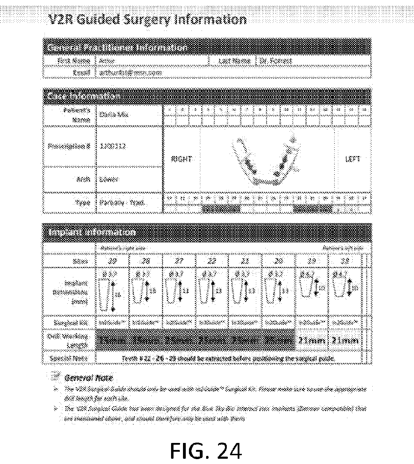

FIG. 24 is a screenshot showing guided surgery information for a patient according to an embodiment of the invention. The surgical guide information may be developed from the CT scans shown previously. The surgical guide information includes a Complete Guided Surgery Protocol including drill selection and handles for preplanned osteotomy position. The surgical guide information includes general practitioner information, case information, and implant information including sites, implant dimensions, surgical kit, drill working length, and special notes.

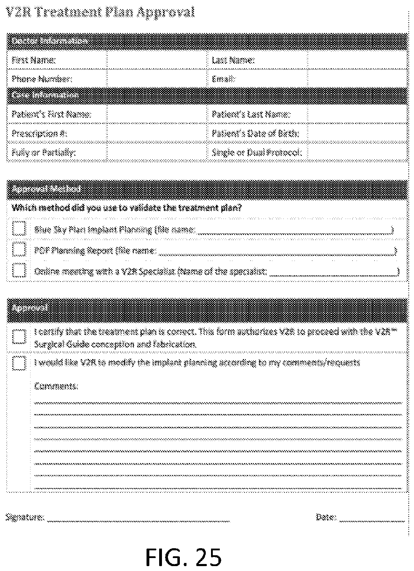

FIG. 25 is a screenshot showing a treatment planning approval form according to an embodiment of the invention. The approval form includes information such as doctor information, case information, and approval method.

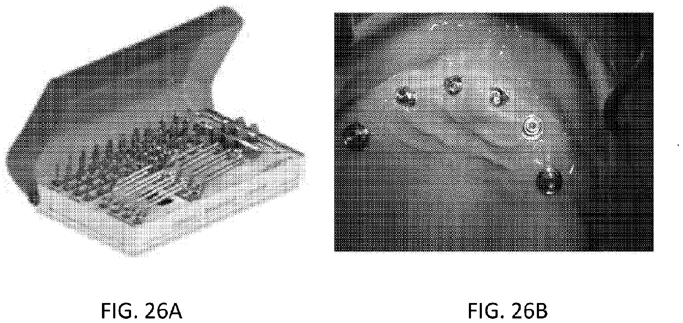

FIG. 26A is a photograph of a guided surgery kit according to an embodiment of the invention. It is an example of the type of guided surgery tools that can be used to drill osteotomies through the drill sites of the surgical guide.

FIG. 26B is a photograph showing fast healing using the guided surgery method. The photographs show the implants placed according to the methods.

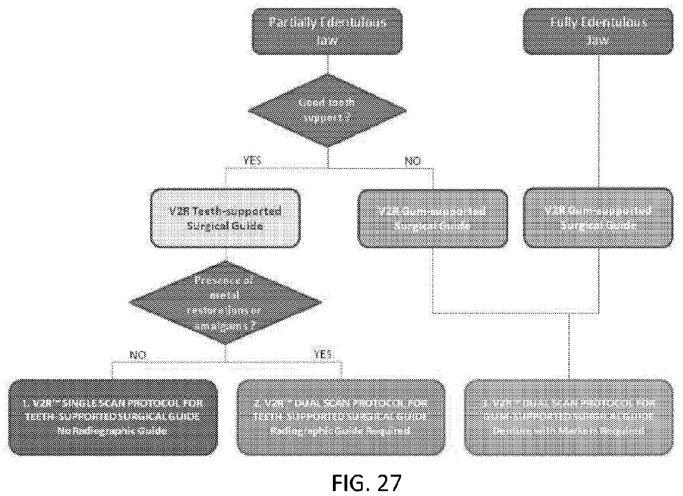

FIG. 27 is a decision tree to support the appropriate scanning protocol according to an embodiment of the invention. The decision tree determines which scanning protocol of FIG. 20 to use. For a partially edentulous jaw, if there is good teeth support, and no metal restorations or amalgams, then the CT scan may be performed without a radiographic guide, and a single scan protocol may be used. If there is good teeth support and amalgams or metal restorations are present, then the CT scan may be performed with a radiographic guide and a dual scan protocol may be used. For a partially edentulous jaw without good teeth support or for a fully edentulous jaw, the CT scan may be performed with a gum-supported surgical guide (denture with markers), and a dual scan protocol for a sum-supported surgical guide may be used.

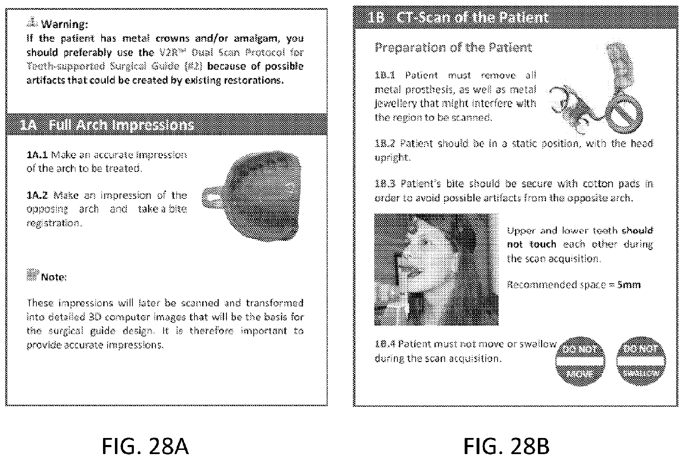

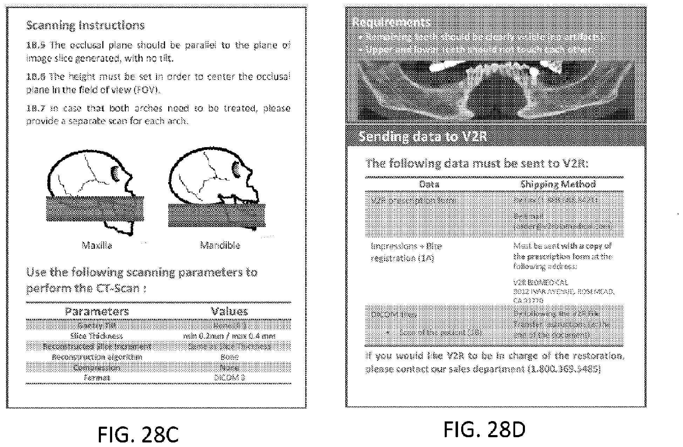

FIGS. 28A-28D are screenshots showing a Single Scan Protocol for Teeth-supported Surgical Guide according to an embodiment of the invention. First, full arch impressions of the arch to be treated and the opposite arch are taken as well as a bite registration. The arch impressions are scanned and transformed into 3D computer images and used as the basis for design of the surgical guide, as shown in FIG. 28A. Then, the patient is prepared according to the instructions in FIG. 28B, and a scan of the patient is performed. A CT scan is then performed according to the scanning instructions and parameters shown in FIG. 28C. The scanning data (DICOM files), impressions, and bite registrations are then sent to a facility and used to create a surgical guide as shown in FIG. 28D.

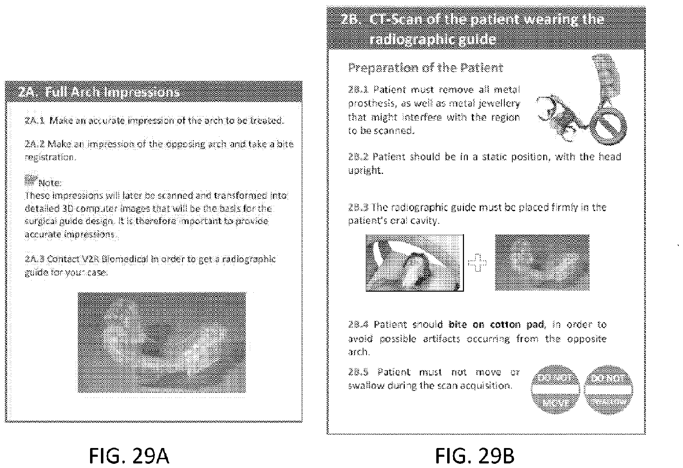

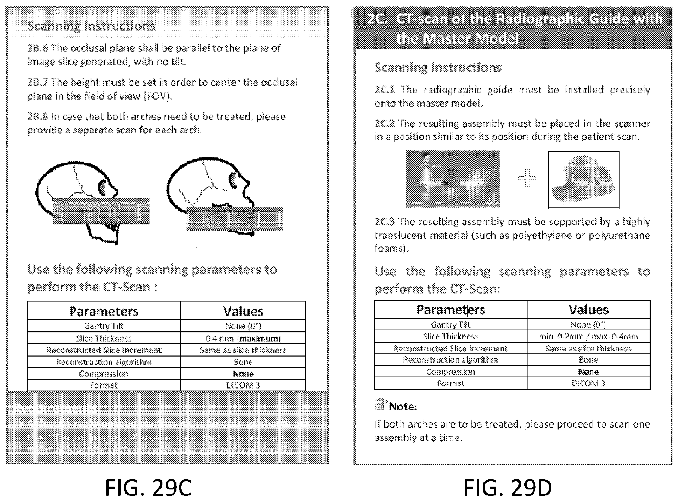

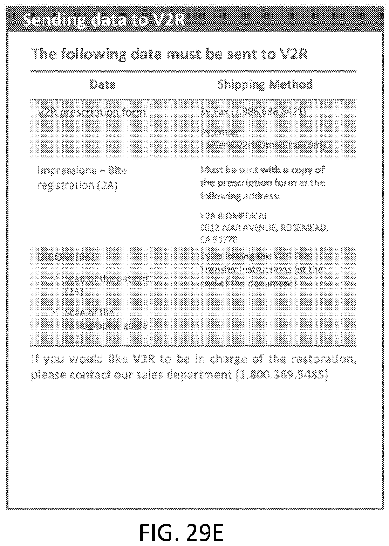

FIGS. 29A-29E are screenshots showing a Dual Scan Protocol for Teeth-supported Surgical Guide according to an embodiment of the invention. The arch impressions are scanned and transformed into 3D computer images and used as the basis for design of the surgical guide, as shown in FIG. 29A. Then, the patient is prepared according to the instructions in FIG. 29B, and a scan of the patient is performed with the radiographic guide in place according to the instructions shown in FIG. 29C. Then, a CT scan of the radiographic guide with the master model obtained from the arch impressions is performed according to the instructions in FIG. 29D. Finally, the scanning data (DICOM files), impressions, and bite registrations are then sent to a facility and used to create a surgical guide as shown in FIG. 29E.

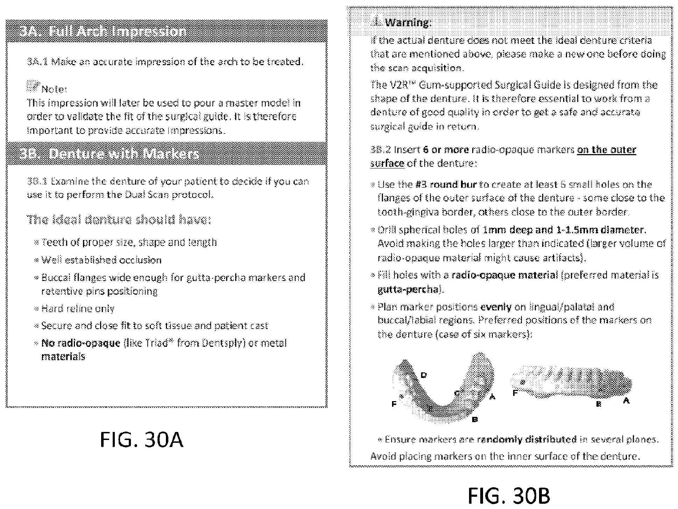

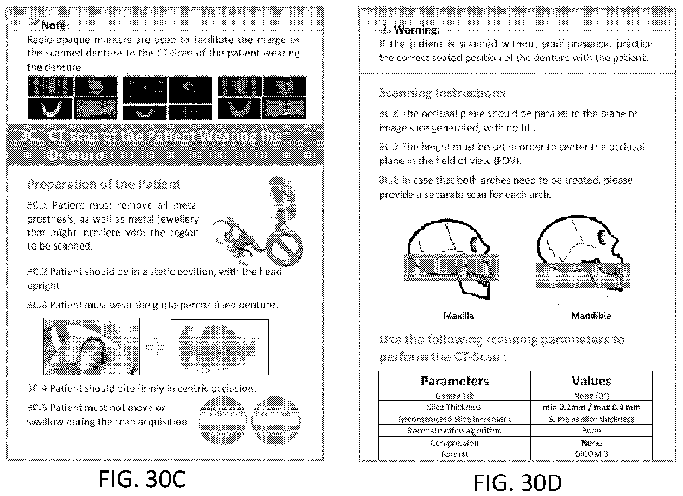

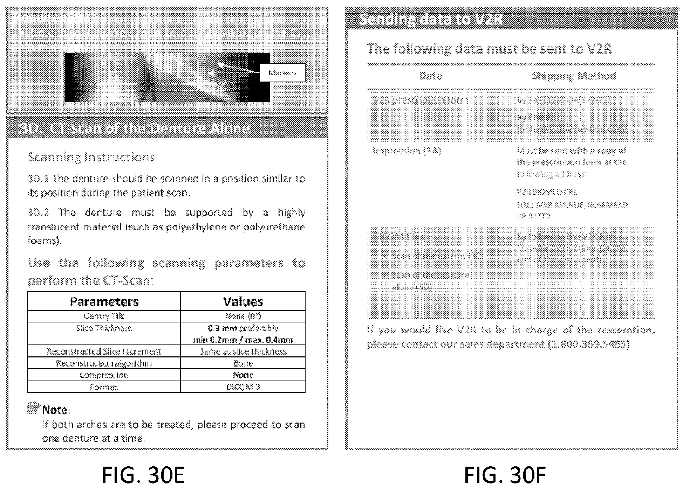

FIGS. 30A-30F are screenshots showing a Dual Scan Protocol for Gum-Supported Surgical Guide according to an embodiment of the invention. Full arch impressions are obtained as shown in FIG. 30A, while a denture with radiopaque markers on the outer surface of the denture is created as shown in FIG. 30B. Then, the patient is prepared for a CT scan wearing the denture according to the instructions in FIG. 30C, and a scan of the patient is performed with the denture in place according to the instructions shown in FIG. 30D. A CT scan of the denture alone is performed according to the instructions in FIG. 30E. Finally, the scanning data (DICOM files) and impressions are then sent to a facility and used to create a surgical guide as shown in FIG. 30F.

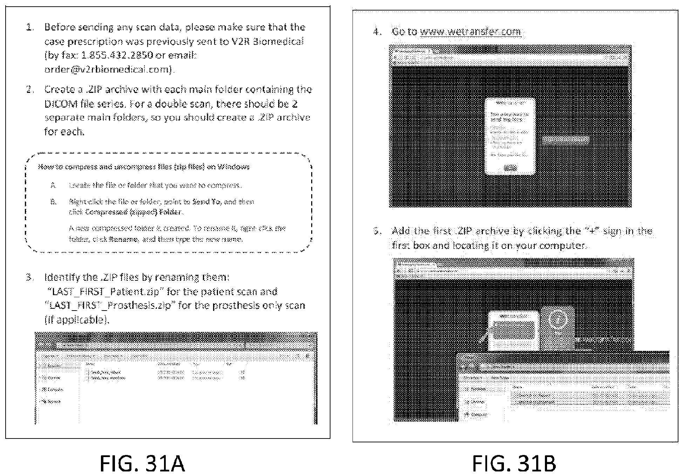

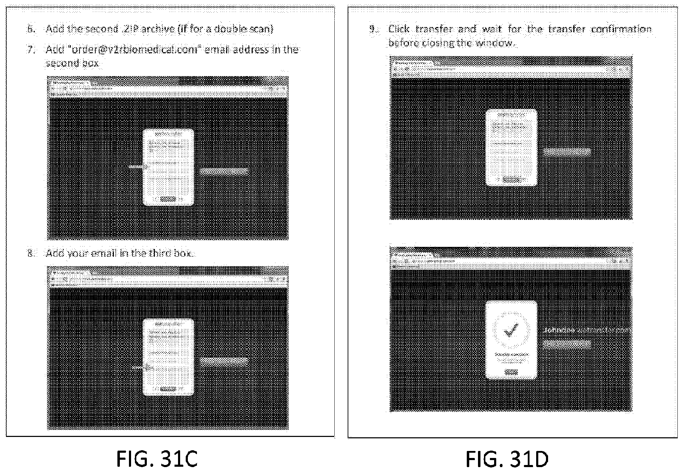

FIGS. 31A-31D are screenshots showing file transfer instructions according to an embodiment of the invention. Such instructions may be used to transfer the DICOM files to a facility where a surgical guide can be created.

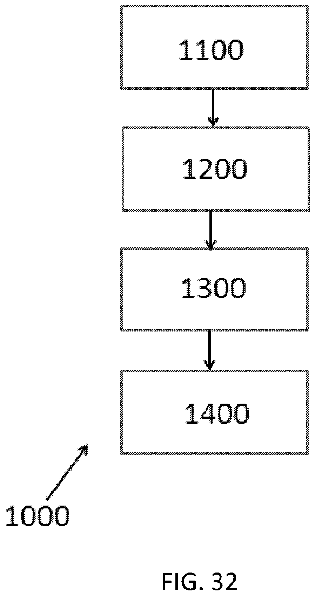

FIGS. 32-34 refer to flowcharts illustrating exemplary methods. FIG. 32 shows an embodiment of a method 1000 for creating a surgical dental template. The method comprises performing a CT scan on a patient 1100, transferring one or more CT scan images into treatment planning software 1200, virtually placing implants in one or more positions on the CT scan using the treatment planning software 1300, and creating a surgical template based on positions on CT scan that provide one or more sites for drilling osteotomies in the maxillary or mandibular jaw for installing the implants 1400.

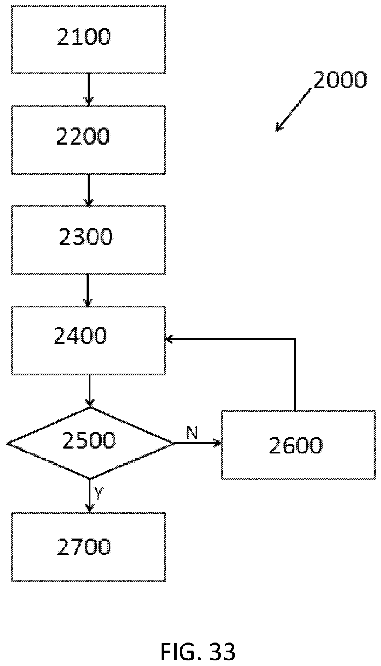

FIG. 33 shows a method 2000 for implanting a denture prosthesis in a partially edentulous patient. The method 2000 comprises providing 2100 a surgical template and a false teeth set of the invention, positioning 2200 the surgical template over the gum tissue of an edentulous jaw of a patient at a first position, inserting 2300 the false teeth set into the surgical template; instructing 2400 the patient to bite down on the false teeth set with natural teeth of the jaw opposite the edentulous jaw, confirming occlusion 2500 with the patient, and if there is no occlusion, repositioning 2600 the surgical template, and repeating until the patient confirms occlusion between the natural teeth and false teeth set; and when occlusion is confirmed, fixing 2700 the surgical template over the gum tissue of the patient's edentulous jaw at a second position.

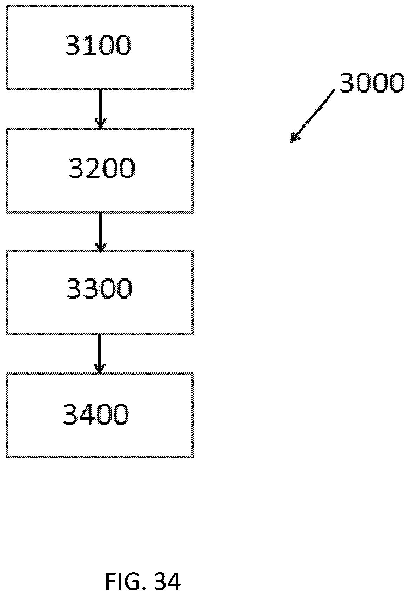

FIG. 34 shows an embodiment of a method 3000 for reducing jaw bone in a patient. The method 3000 comprises installing 3100 a surgical template of the invention, wherein the surgical template has one or more sets of drill holes, perforating osteotomies 3200 through the drill holes in the surgical template to define markings in the jaw bone forming a boundary for reducing bone, removing the surgical template 3300 and exposing the jaw bone though an incision to reveal the osteotomies, and using a surgical instrument to remove jaw bone 2400 based on the boundary formed by the markings.

Another embodiment of the invention provides a guided pin system for immediate load consists of a series of guides that reference a first set of fixation pins throughout the surgical and prosthetic protocol. All sequential guides thereafter will share these same fixation site reference points. In embodiments, the system of sequential guides comprises 1) a tooth and tissue borne surgical guide with fixation pins and bone reduction osteotomy sites (also referred to herein as a "first guide") 2) an implant osteotomy guide (also referred to herein as a "second guide" and 3) a prosthesis guide (also referred to herein as a "third guide"). The system does not rely on any anatomical reference points such as tissue or bone after placement of the first tooth and tissue borne reference guide. The sequential guide(s), both surgical implant placement and prosthesis guide rely on the original fixation positions determined by the first guide. Digital treatment planning and design are used to accurately place each sequential guide back to the original fixation pin positions. The fixation pin reference can be pinned and repinned in the same sites per sequential guide or fixed in the jaw through the protocol by keeping them in place and switching out the guides.

In embodiments, the first, second, and third guide are each configured to fit a dental surgical site of a patient. The dental surgical site may be a portion of a partially edentulous maxillary or mandibular jaw, such as the portion inside the mouth that supports the natural teeth and/or gum tissue, such as shown in the following figures. In embodiments, the first dental surgical guide may be configured so that it aligns and is oriented at the dental surgical site through one or more teeth of the patient, which may be natural teeth or implants. The second dental surgical guide is configured to allow for drilling of osteotomies for placement of implants, or abutments, at the dental surgical site. These abutments will be used to secure the third guide in place. The third dental surgical guide may be configured to provide for the appearance of natural teeth such that the third dental surgical guide may serve as a dental prosthesis. In embodiments, the first, second, and third dental surgical guides each comprise one or more anchoring sites for attachment to jaw bone of the patient at the dental surgical site. Because the anchoring sites occupy the same relative positions on each guide, each of the guides may be secured by way of a fastener such as a pin through a single set of osteotomies that are drilled through the anchoring sites of the first guide. Thus, the first guide provides a reference set of osteotomies for fixation pins (or similar fastener) to provide a basis for alignment and fixation of subsequent dental surgical guides of the system.

Embodiments of the invention include a system with three sequential surgical guides; however, systems that include additional surgical guides that operate on the same principle of alignment also fall within the scope of the invention.

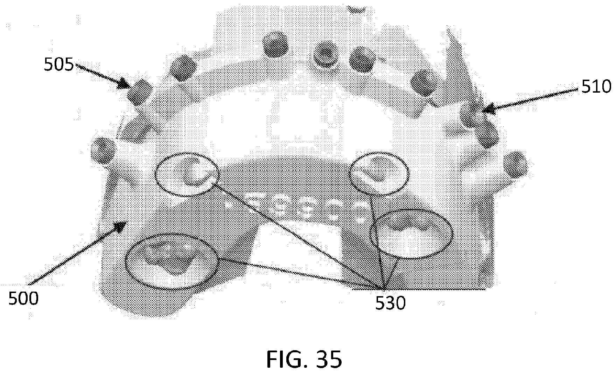

The following figures will further illustrate embodiments of the system of the invention. FIG. 35 shows an embodiment of a tooth-borne surgical guide, or first guide 500. Shown at the top of the guide are six bone reduction sites 505 and four anchoring sites 510. The bone reduction sites 505 and anchoring sites 510 each provide a passage or channel for drilling osteotomies at a precise location during the surgical procedure. Additionally, the anchoring sites 510 provide for passage of fastener such as a pin for fixing the guide in place. Also shown are inspection windows or voids 530 for aligning the surgical guide with existing teeth (e.g. natural or artificial teeth (implants)) in the partially edentulous jaw for accurate positioning of the first guide 500. The first guide 500 can be produced based on a CT scan of the patient's jaw, which allows a technician to design the guide according to existing teeth as landmarks in the patient's mouth. Thus, the first guide 500 fits like a puzzle piece in the patient's mouth as a result of the placement of the inspection windows 530 according to the locations of existing teeth in the partially edentulous jaw. Most importantly, the tooth-bome surgical guide 500 sets the stage for the rest of the surgical procedure by providing the anchoring sites 510, which provide for reference points in the jaw based on osteotomies drilled through the anchoring sites for subsequent guides, and the bone reduction sites 505 which guide the placement of osteotomies which will be used to define the amount of bone reduction necessary for proper placement of the second guide (osteotomy guide) and third guide (prosthetic guide).

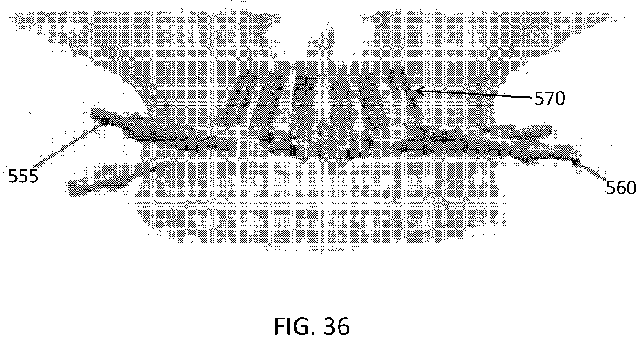

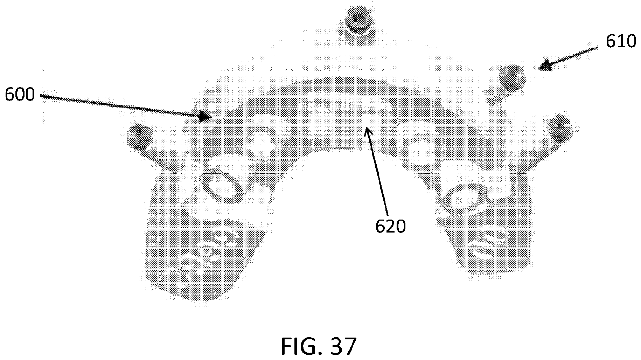

FIG. 36 shows a bone model of a patient showing bone reduction pins 555, anchoring pins 560, and implants 570 based on placement of the guide of FIG. 35. These represent the sites that will be drilled during the surgical procedure. The next figure, FIG. 37, shows an embodiment of the implant osteotomy guide, or second guide 600. Shown in the figure are the anchoring sites 610 which are in the same relative positions on the guide as the anchoring sites 510 of the first guide. The anchoring sites 610 allow the implant osteotomy guide to be precisely placed in the mouth for correct placement of the implants and securing in the jaw through a pin or other fastener. Also shown are the osteotomy drilling sites 620 for the implants with depth stops for limiting the depth of the osteotomies to a specific target depth. Such osteotomy sites 620 and depth stops are also determined through CT scan imaging of the patient.

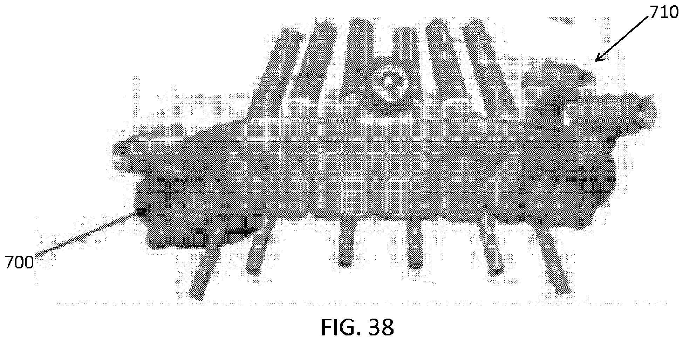

FIG. 38 shows the last of the sequential guides, or third guide, which is the prosthetic guide 700, and shows the abutments, screw channel axis, and the anchoring sites 710 which are also in the same relative positions on the guide as the anchoring sites 510 of the first guide and operate to provide a passage for a pin or other fastener for securing the guide in place. The third guide will serve as the final prosthesis.

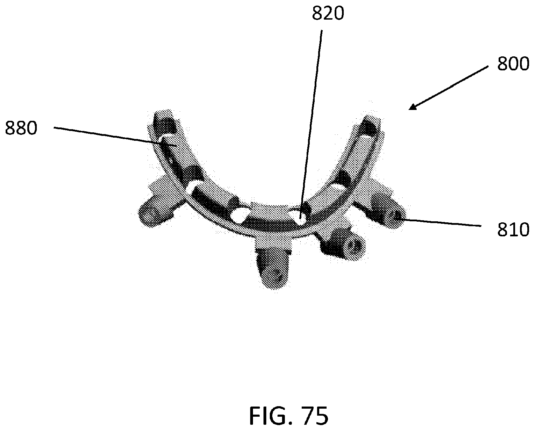

Now referring to FIG. 75, in another embodiment, a superstructure/bar 800 can be designed to fit the anchoring pins 560 installed use of the first guide 500. The superstructure 800 will take the place of the mandibular guide 105 or maxillary guide 125. The superstructure 800 is made to fit the drill sites 120 with its own drill sites 820. The superstructure 800 can be fashioned to act as the osteotomy drill guide. When used as an osteotomy guide, a depth component will eliminate the need for the implant osteotomy drill guide, or second guide. In another embodiment, the superstructure can be used after implant osteotomy drill guide 600 if a depth component is not incorporated. Depending on the angulation and other factors of the implant positions, the false teeth sets 112, 118 can be attached to the superstructure 800. The superstructure 800 is manufactured using biocompatible material such as titanium, Peek, Fiber or like materials used to fabricate dental superstructures/bars.

The superstructure 800 mates with false teeth sets 112, 118 directly via a ledge 830. Alternatively, the superstructure 800 can be made with protrusions to provide more surface area for mating with a false teeth set.

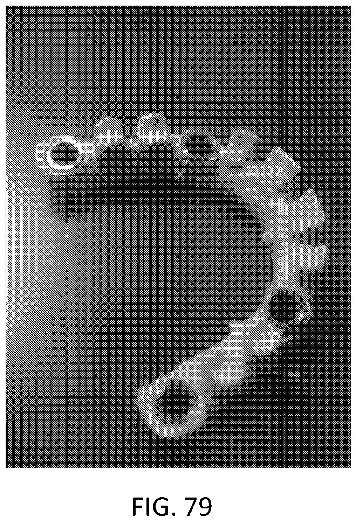

Now referring to FIG. 79, in another embodiment, the superstructure has teeth shaped protrusions. The false teeth attach to the superstructure with corresponding teeth shaped cutouts. This embodiment provides more surface area for adhering the false teeth to the superstructure.

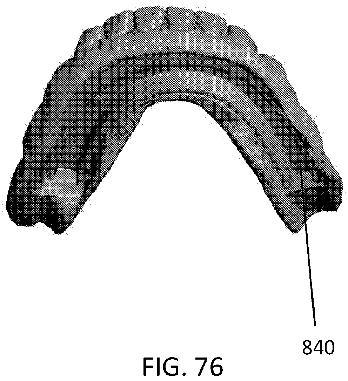

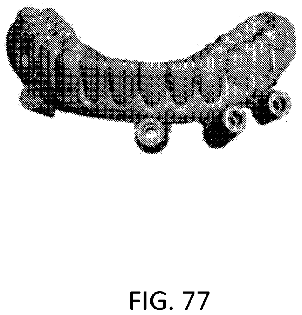

Now referring to FIG. 76, the mandibular false teeth 112 have a groove 840 that is made to match the ledge 830 of the superstructure. FIG 77 shows the superstructure 800 and mandibular false teeth 112 mated together via the groove of the false teeth and the protrusion of the superstructure.

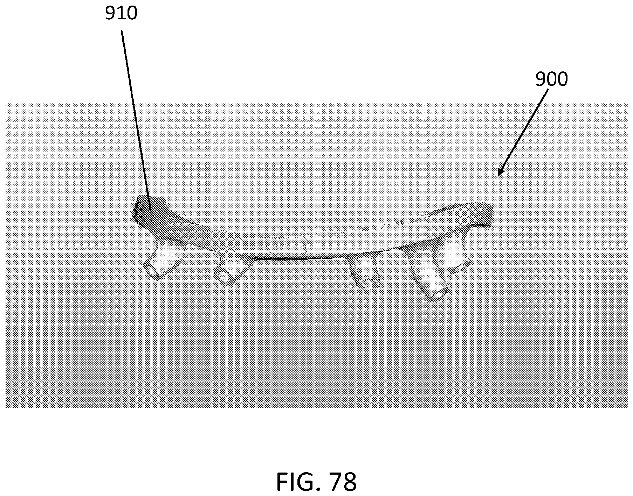

FIG 78. shows a bone reduction jig 900. The jig uses the same anchoring pins 560 installed with the first guide 500. Once anchored in place, the jig is used a guide for reducing bone to the depth required for installing the implants. The bone reduction jig 900 eliminates the need of the first guide's bone reduction sites 505, and allows for more precise bone reduction as compared to using bone reduction pins 555. A ridge 910 on the bone reduction jig allows for bone reduction to be more accurate. Osteotomies are more precise and accurate with the jig.

The next set of figures is intended to illustrate the use of the sequential guide system in an actual surgical procedure. While these figures are intended to demonstrate the method of use of the sequential guide system as it occurs during a surgical procedure, examples of the disclosure include variations in the order of the steps shown in the figures.

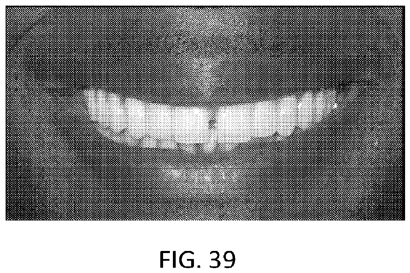

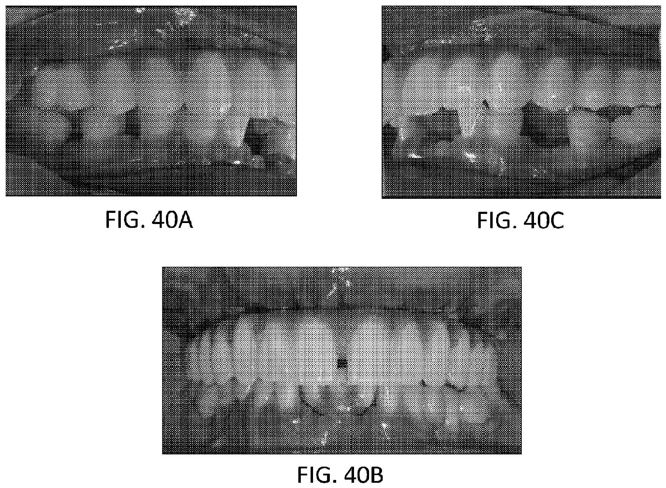

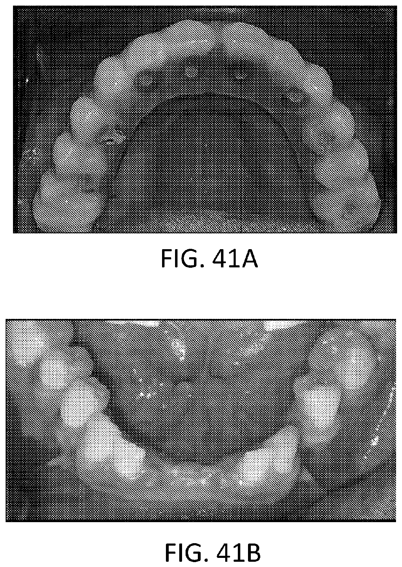

Turning now to FIG. 39, a partially edentulous mouth of a patient is shown. The patient has had a prosthesis placed on the maxillary jaw. The mandibular jaw still has some remaining teeth. This is shown more clearly in FIGS. 40A-40C. FIG. 41A shows a bottom view of the maxillary implant, while FIG. 41B shows a top view of the partially edentulous mandibular jaw of the patient.

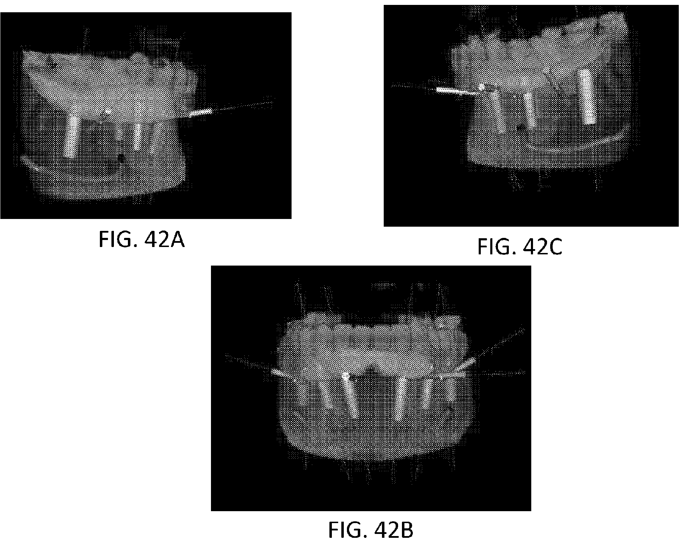

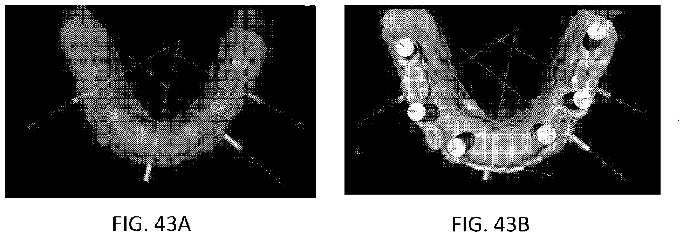

FIGS. 42A-42C show a CT treatment plan for the patient. Shown are representations of the six implant sites as well as the four anchoring sites. These sites will be used for placement of osteotomies in the patient's jaw. FIGS. 43A and 43B show an occlusal view of the CT treatment plan. The anchoring sites and screw channel position and axis are shown. The CT treatment plan may determine the exact positioning of the implant sites, anchoring sits, bone reduction sites, and the placement of osteotomies based on features identified in the CT scan. These features may include sites of relative bone density, the presence of blood vessels or nerves, or other features identified in the CT scan.

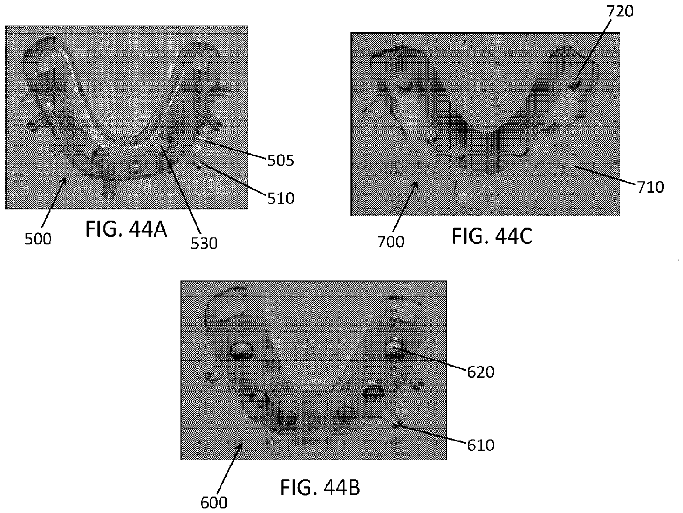

FIGS. 44A-44C show a top view of the three sequential guides placed on a flat horizontal surface, with FIG 44A showing the tooth-supported surgical guide 500 with bone reduction sites 505 and anchoring points 510, FIG 44B showing the implant guide 600 with anchoring points 610 occupying the same relative positions as the anchoring points 510 on the tooth-supported surgical guide 500, and FIG. 44C showing the prosthetic guide 710 with anchoring points 710 occupying the same relative positions as anchoring points 510 of first guide 500. The anchoring points are placed in the same positions so that the sequential guides 500, 600, 700 can be secured through the same set of osteotomies in the jaw. Securement may be achieved through a set of fasteners, such as pins placed in the osteotomies determined by the anchoring points 510 of the first guide 500.

Further, it can be seen from the top view provided by FIGS. 44A-44C that anchoring sites 510, 610, and 710 provide channels which are oriented substantially horizontal on the three sequential guides 500, 600, 700. However, some of the channels provided by the anchoring sites 510, 610, and 710 may deviate from horizontal in either direction by 1, 2, 3, 4, 5, 10, 15, 20, 25, 30, 35, 40, 45 degrees or more. Bone reduction sites 505 on first guide 500 may also be oriented substantially horizontal and may provide for similar deviation from horizontal. Further, as seen in FIGS. 44A-44C, voids 530, 620, 630 are present which are oriented substantially vertical on the three sequential guides 500, 600, 700. However, such voids 530, 620, 720 may also deviate from vertical by 1, 2, 3, 4, 5, 10, 15, 20, 25, 30, 35, 40, 45 degrees or more. However, on average, anchoring sites 510, 610, 710 are oriented relatively orthogonal to voids 530, 620, 720 on sequential guides 500, 600, 700 and vice versa. Similarly, bone reduction sites 505 are oriented relatively orthogonal to voids 530 on sequential guide 500. The exact orientation of each of the anchoring sites 510, 610, 710, bone reduction sites 505, and voids 530, 620, 720 will be determined by the results of the CT scan used for treatment planning and will be based on features of the patient's jaw at the dental surgical site identified in the CT scan, such as sufficient bone density, the location of blood vessels and nerves, etc.

Additionally, channels provided by anchoring sites 510, 610, 710, bone reduction sites 505, and voids 530, 620, 720 may be any shape. A circular shape of anchoring sites 510, 610, 710 and bone reduction sites 505 allows for passage of a fastener such as a pin. Embodiments of such sites may have non-circular shapes as well, including oval, square, triangular, or other polygon. Similarly, voids 530, 620, 720 may have any polygonal or non-polygonal shape. Further, channels provided by anchoring sites 510, 610, 710 are typically just large enough to allow passage of a fastener on the order of 1-3 millimeters in diameter, while bone reduction sites are smaller, allowing passage of a thin bore drill bit. In contrast, voids 530, 620, 720 are considerably larger, spanning 0.5 cm to 1 cm or greater in diameter, as voids 530 are configured to accommodate teeth, while voids 620, 720 are configured to allow drilling for implant of abutments.

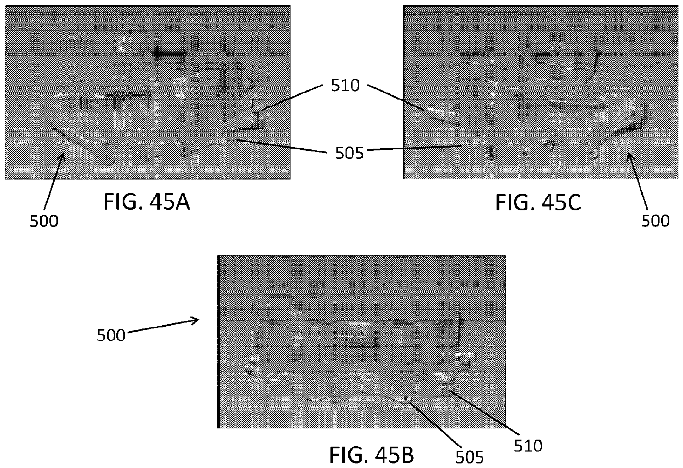

FIGS. 45A-45C show different views of the tooth-supported surgical guide 500. Shown in the figures are the bone reduction sites 500 and anchoring points 510.

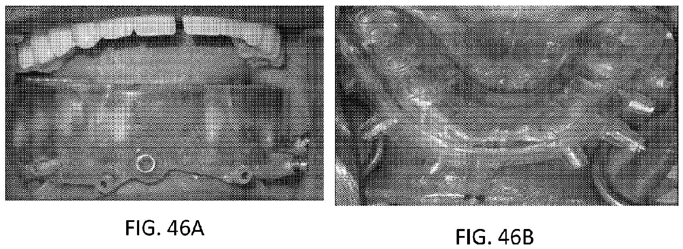

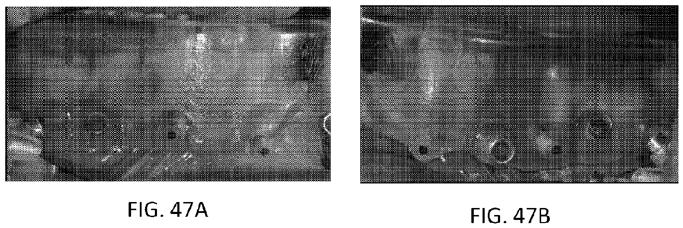

Now, turning to FIGS. 46A and 46B, shown is the beginning of the procedure with placement of the tooth-supported surgical guide at the surgical site. FIGS. 47A and 47B show fit verification of the tooth-supported surgical guide. As shown in the figure, the tooth-supported surgical guide fits like a puzzle piece over the existing teeth in the partially edentulous jaw.

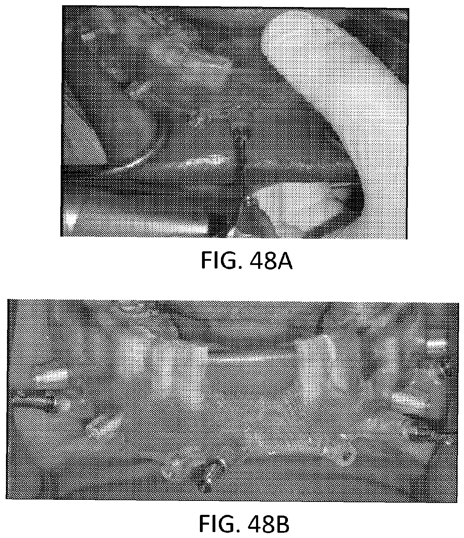

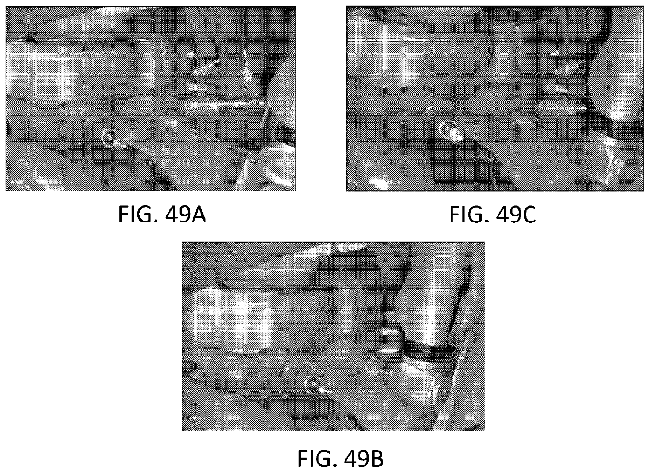

FIG. 48A shows the drilling of osteotomies for the anchoring sites, while FIG. 48B shows fixation of the guide in the jaw through the placement of fixation (or anchoring) pins. In the next step in the procedure (shown in FIGS. 49A-49C), horizontal osteotomies are drilled through the channels provided by the bone reduction sites.

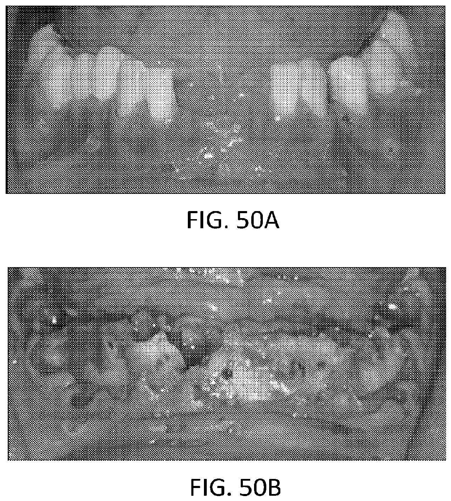

In the next step, shown in FIGS. 50A and 50B (before and after), once the bone reduction osteotomies and anchoring osteotomies have been drilled, the remaining are extracted and the gum tissue is cut open and flapped to expose the jaw bone and drilling sites.

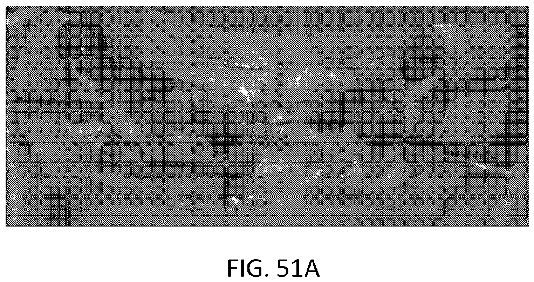

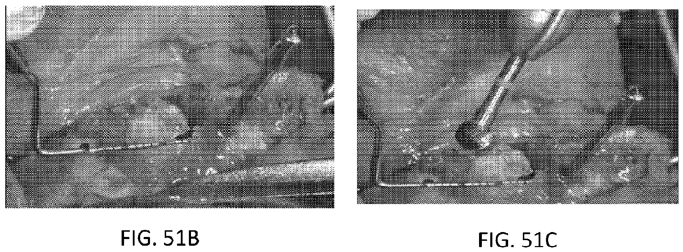

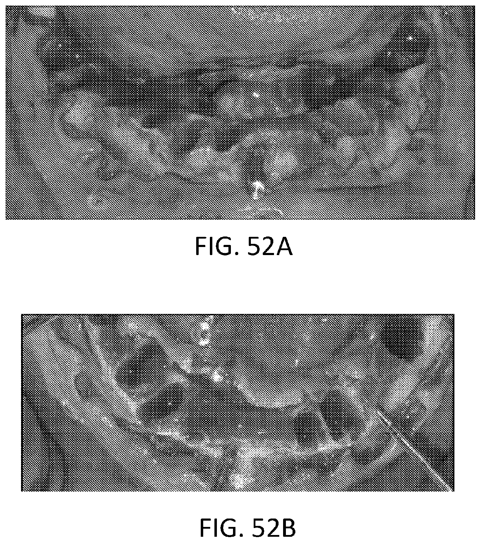

FIG. 51A shows the guide pins reinserted into the jaw bone for visual purposes, while FIGS. 51B and 51C show identification of the bone reduction sites and bone removal around the arch through a "connect the dots" method. Complete bone reduction is shown in FIGS. 52A and 52B.

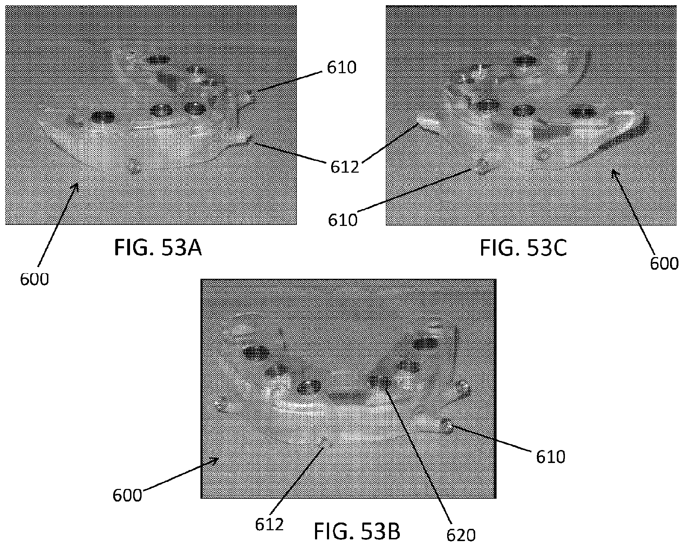

FIGS. 53A-53C show an embodiment of an implant osteotomy guide 600, which will be used in the next step in the procedure. Shown in the figures are anchoring sites 610 and osteotomy sites 620 for implants. Also shown is open channel anchoring site 612, which will be used in the surgical procedure in combination with one of the guide pins as a reference point, as described below. However, in other embodiments, implant osteotomy guide 600 may have more than one open channel anchoring site 612, including 2, 3, 4, or more. As seen in FIG. 53B, open channel anchoring site 612 is in communication with the bottom edge of the implant osteotomy guide.

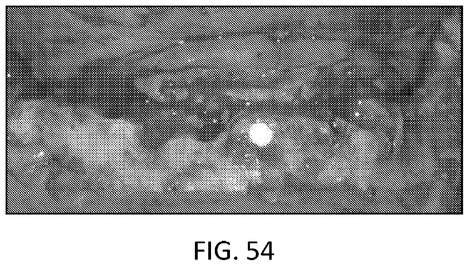

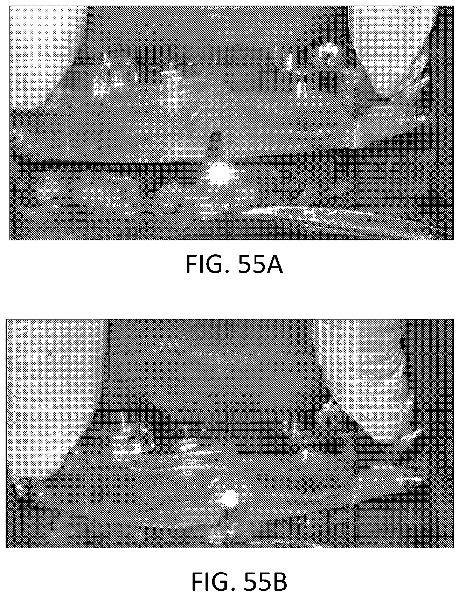

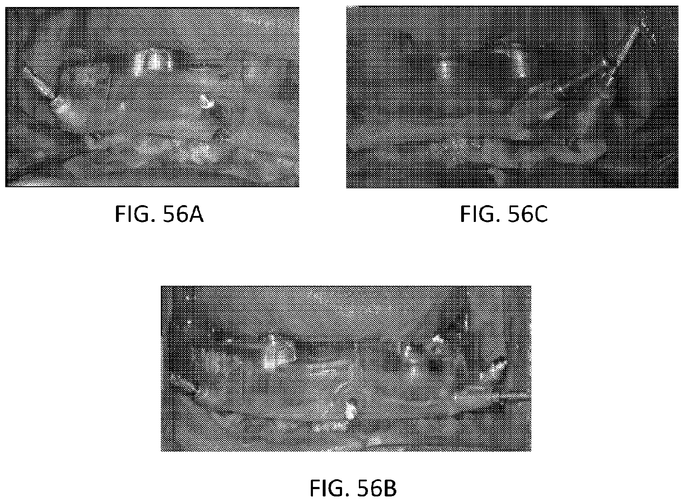

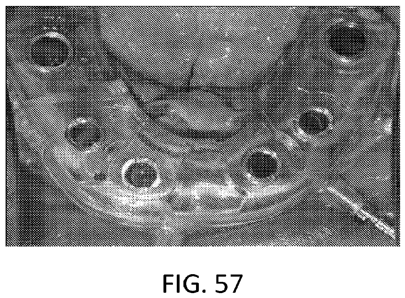

Once bone reduction is complete, the surgical site is ready for the implant osteotomy guide (second guide). First, one of the insertion pins is placed in jaw bone to provide a reference point, as shown in FIG. 54. This insertion pin corresponds to the channel anchoring site in the osteotomy guide which has a portion in communication with the bottom edge of the guide such that the guide can reference this pin and be placed over it from a vertical approach. As can be seen in FIGS. 55A and 55B, this channel is aligned to the insertion pin during vertical placement of the guide, so that the guide is properly orientated in the jaw. Then, as shown in FIGS. 56A-56C, the remaining anchoring sites are pinned to secure the implant osteotomy guide in the jaw. The insertion pins will be placed in the original osteotomy sites that were determined by the first guide. FIG. 57 shows the osteotomy guide installed.

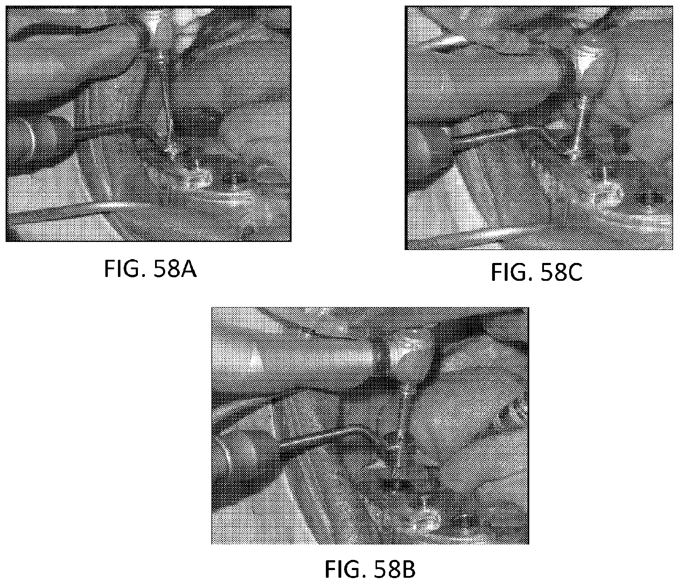

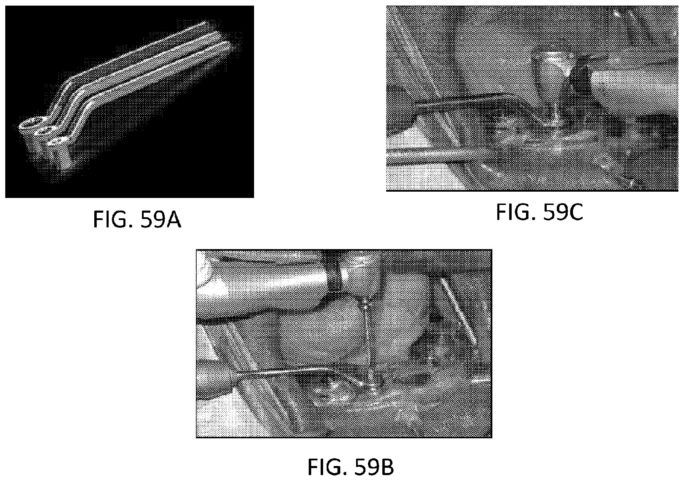

Once the implant osteotomy guide is installed, osteotomies are performed based on the implant manufacturer's guided protocols. Drilling of osteotomies is shown in FIGS. 58A-C. FIG. 59A shows a tool for performing osteotomies. FIGS. 59B and 59C show additional drilling of osteotomies.

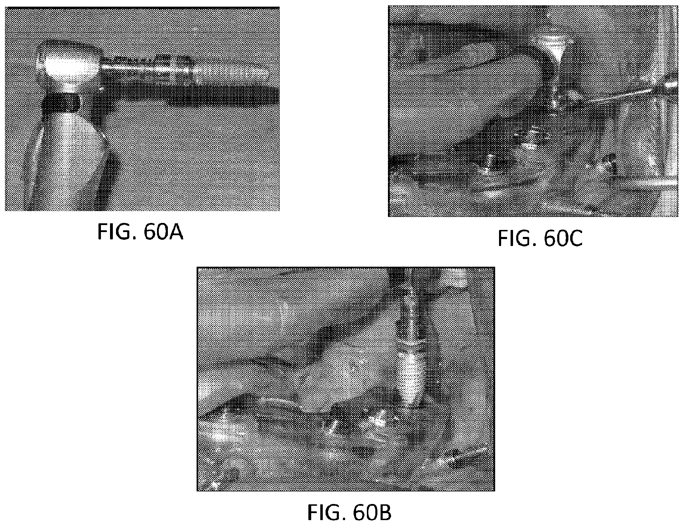

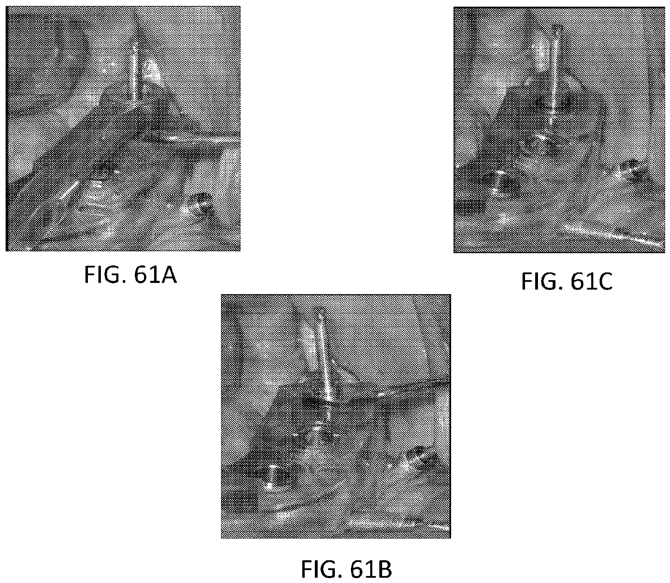

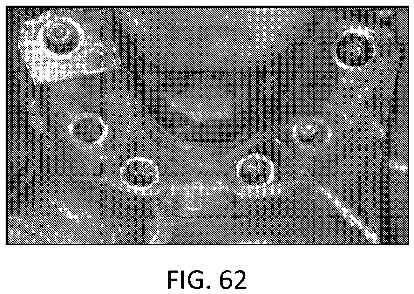

Once the osteotomies are drilled, the implants may be installed in the jaw according to the implant manufacturers guided protocol. FIG. 60A shows a drill for installing implants, and FIGS. 60B and 60C show installation of implants into the drilled osteotomies. FIGS. 61A-61C show implants being placed with a driver. FIG. 62 shows the implants fully seated.

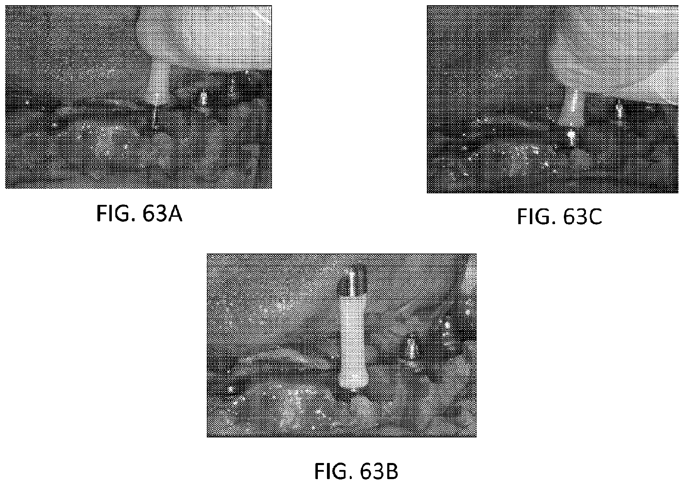

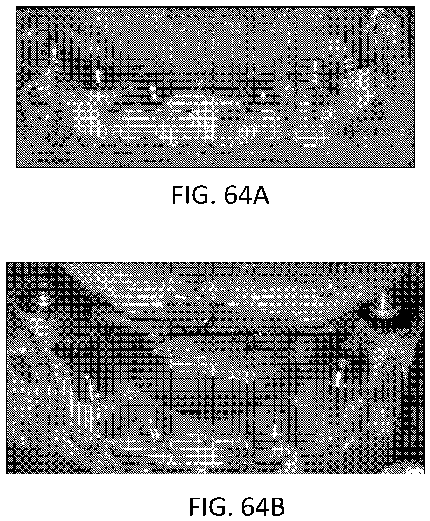

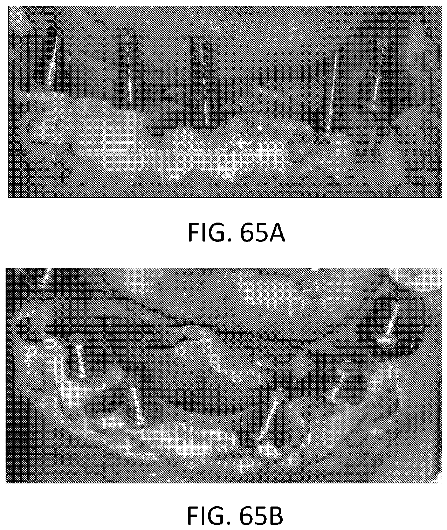

Once the implants are fully seated, the abutments may be installed. FIGS. 63A-63C show insertion of multi-unit abutments (pre-determined guided hex position with screw channel orientation), while FIGS. 64A and 64B show the abutments installed. After installation of the abutments, temp copings are installed on top of the abutments; this is shown in FIGS 65A and 65B. The temp copings will be picked up by the third guide (the prosthetic guide).

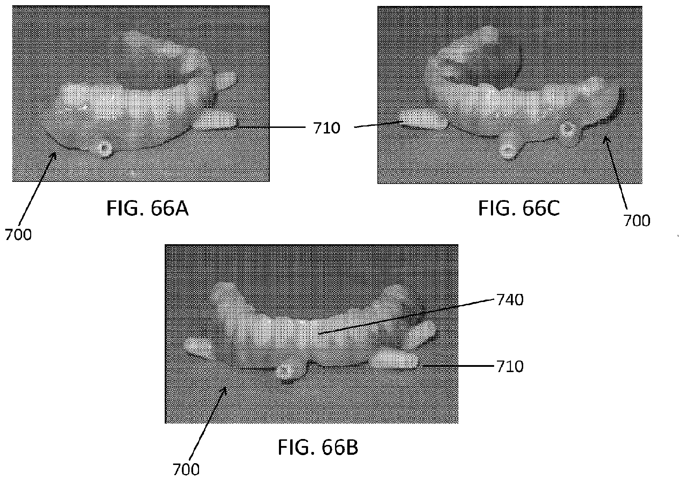

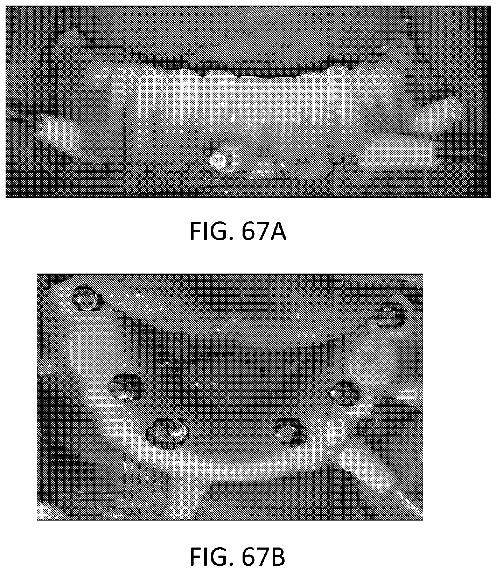

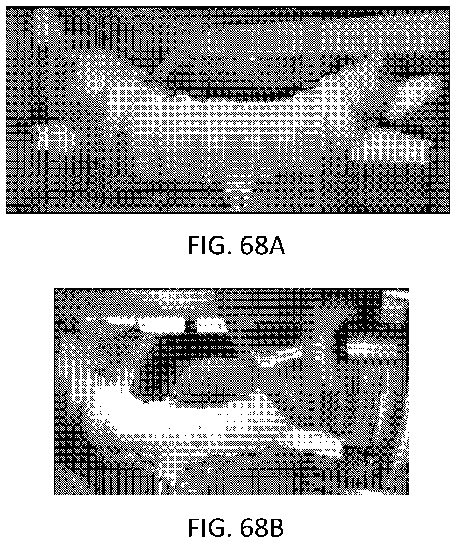

After installation of the temp copings, the final phase of the procedure, installation of the third guide, is performed. FIGS. 66A-66C show various perspective views of the third guide (prosthetic guide) 700. Shown in the prosthetic guide are the anchoring sites 710. Also shown are prosthetic teeth 740 which simulate the appearance of natural teeth. FIGS. 67A-67B show placement of the third guide in the lower jaw. As can be seen from FIGS. 67A-67B, the third guide fits such that the temp copings fit in the holes of the third guide, and the third guide is pinned in place using the same osteotomies determined from the anchoring sites present in the first guide. FIGS. 68A and 68B show that the pinned prosthesis ensures preplanned midline and occlusal reference and also shows filling of holes in the guide with light-cured composite.

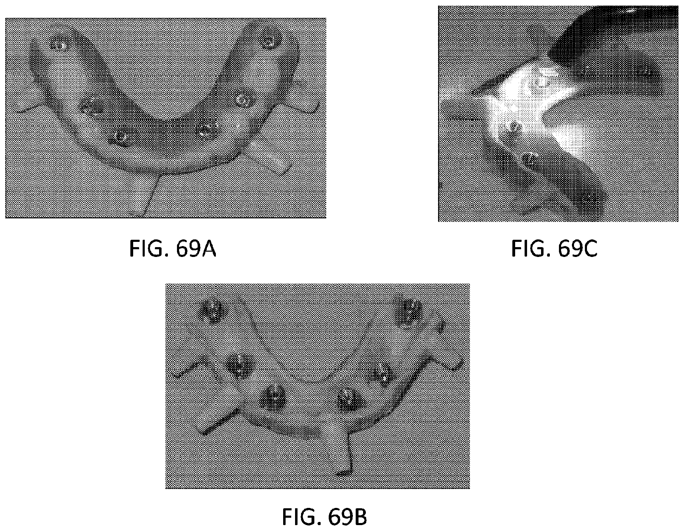

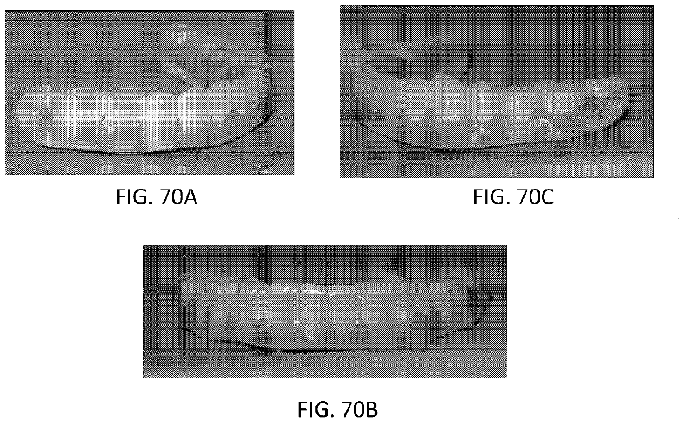

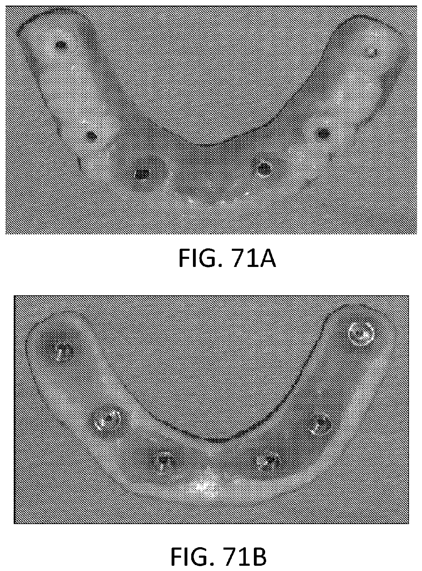

The prosthesis guide is then removed from the mouth and processed for conversion to the final prosthesis. FIGS. 69A-69C show that voids in the prosthesis are filled with light-cured composites or acrylics outside the mouth. FIGS. 70A-70C show different perspective views of the final prosthesis with insertion pins and pin holes trimmed, flanges reduced, and convex intaglio. FIGS. 71A and 71B show top and bottom images of the final prosthesis.

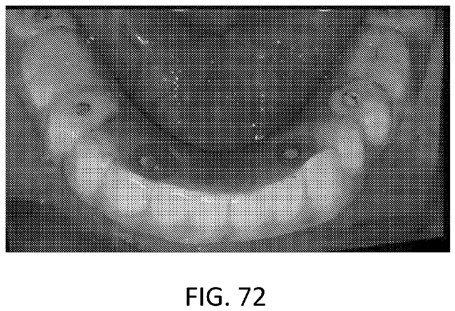

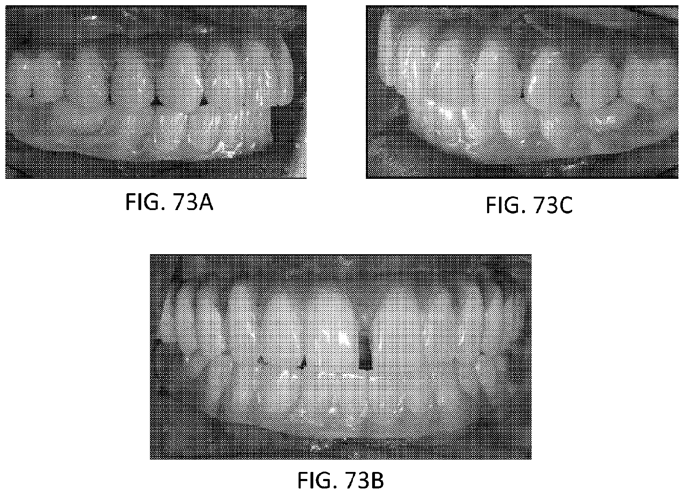

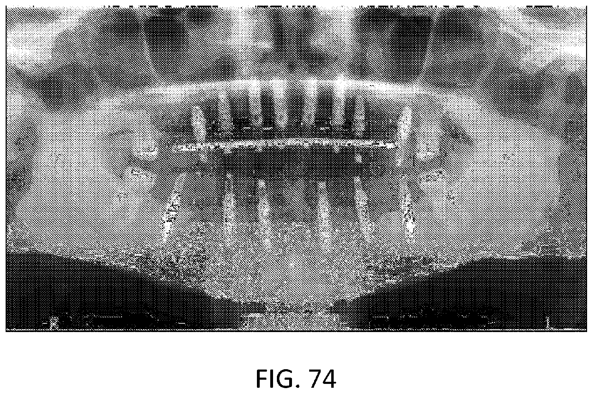

FIG. 72 shows the final mandibular prosthesis in the mouth. FIGS. 73A-73C show occlusion of the top and bottom prostheses. FIG. 74 shows an X-ray image of the patient, which shows placement of the implants.

Other embodiments of the system are envisioned which may deviate from the description and figures provided above but which fall within the scope of the invention. For example, in other embodiments, the fixation pins may remain in the osteotomies throughout the surgical procedure as reference points for attaching subsequent guides. The pins may be shaped so that each guide can be fitted at the dental surgical site over the pins at the anchoring sites while the pins are still in place. Alternatively or in addition, the surgical guides may include channels that are open to the bottom edge for vertical placement of the guides as shown in FIG. 53B (feature 612). Additionally, another embodiment comprises a two-part pin system, in which a first portion of a pin remains in osteotomies as a reference point for attaching subsequent guides (the guides fit over this portion), which a second portion of the pin attached once the guide is in place to finish securing the guide in place. The second portion of the pin may be wider than the first portion and the channels of the anchoring sites to prevent removal of the guide.

## Claims

1. A dental surgery system for implanting a prosthesis, comprising:
a first dental surgical guide (500), a second dental surgical guide (600), and a third dental surgical guide (700) each configured to fit a dental surgical site of a patient, wherein the dental surgical site is a portion of a partially edentulous maxillary of mandibular jaw of the patient, wherein:
the first dental surgical guide (500) is configured to align with one or more teeth of the patient at the surgical site;
the second dental surgical guide (600) is configured to allow for drilling of osteotomies for placement of implants at the surgical site;
the third dental surgical guide (700) is configured to provide for the appearance of natural teeth such that the third surgical guide (700) may serve as a dental prosthesis; and
the first dental surgical guide (500), second dental surgical guide (600), and third dental surgical guide (700) each comprise one or more anchoring sites (510, 610, 710) for attachment to jaw bone of the patient, wherein the anchoring sites (510, 610, 710) occupy the same relative positions on the first, second, and third dental surgical guides (500, 600, 700) such that the guides (500, 600, 700) may be aligned and secured by a single set of osteotomies determined by the anchoring sites (510, 610, 710) of the first dental surgical guide (500).

2. The dental surgery system of claim 1, wherein the first dental surgical guide (500) comprises one or more voids (530) corresponding to the positions of the one or more teeth.

3. The dental surgery system of claim 1, wherein the second surgical guide (600) comprises one or more voids (620) which provide a passage for drilling of osteotomies for placement of implants at the dental surgical site.

4. The dental surgery system of claim 3, wherein the third surgical guide (700) comprises one or more voids (720) which occupy the same relative positions as the one or more voids of the second dental surgical guide (600).

5. The dental surgery system of claim 1, wherein the third surgical guide (700) comprises one or more features resembling natural teeth.

6. The dental surgery system of claim 1, wherein the one or more anchoring sites (510, 610, 710) are configured to provide a channel for passage of a fastener through each of the first, second, and third dental surgical guides (500, 600, 700).

7. The dental surgery system of claim 1, wherein the first dental surgical guide (500) comprises one or more bone reduction sites (505) configured to provide for the passage of a drill bit.

8. The dental surgery system of claim 7, wherein the one or more bone reduction sites (505) comprise a linear array of at least three channels positioned horizontally along the first dental surgical guide (500).

9. The dental surgery system of claim 8, wherein the at least three channels provide a template for at least three osteotomies which map a boundary for reducing bone in the jaw.

10. The dental surgery system of claim 1, wherein at least one of the anchoring sites (610) on the second surgical guide (600) is a channel configured for alignment with and placement of the second surgical guide (600) over a reference fastener placed at the surgical site.

11. The dental surgical system of claim 10, wherein the channel is in communication with a bottom edge of the second surgical guide (600).

## Patentansprüche

1. Zahnchirurgisches System zum Implantieren einer Prothese, mit:
einer ersten zahnchirurgischen Führung (500), einer zweiten zahnchirurgischen Führung (600) und einer dritten zahnchirurgischen Führung (700), die jeweils derart konfiguriert sind, dass sie einer zahnchirurgischen Stelle eines Patienten angepasst sind, wobei die zahnchirurgische Stelle ein Abschnitt eines partiell zahnlosen Oberkiefers oder Unterkiefers des Patienten ist, wobei:
die erste zahnchirurgische Führung (500) dafür konfiguriert ist, mit einem oder mehreren Zähnen des Patienten an der zahnchirurgischen Stelle ausgerichtet zu werden;
die zweite zahnchirurgische Führung (600) dafür konfiguriert ist, Osteotomien zum Platzieren von Implantaten an der zahnchirurgischen Stelle zu bohren;
die dritte zahnchirurgische Führung (700) dafür konfiguriert ist, das Aussehen natürlicher Zähne zu gewährleisten, so dass die dritte zahnchirurgische Führung (700) als eine Zahnprothese dienen kann; und
die erste zahnchirurgische Führung (500), die zweite zahnchirurgische Führung (600) und die dritte zahnchirurgische Führung (700) jeweils eine oder mehrere Verankerungsstellen (510, 610, 710) zur Befestigung am Kieferknochen des Patienten aufweisen, wobei die Verankerungsstellen (510, 610, 710) die gleichen relativen Positionen auf der ersten, zweiten und dritten zahnchirurgischen Führung (500, 600, 700) einnehmen, so dass die Führungen (500, 600, 700) durch einen einzelnen Satz von Osteotomien, die durch die Verankerungsstellen (510, 610, 710) der ersten zahnchirurgischen Führung (500) bestimmt sind, ausgerichtet und gesichert werden können.

2. Zahnchirurgisches System nach Anspruch 1, wobei die erste zahnchirurgische Führung (500) einen oder mehrere Hohlräume (530) aufweist, die den Positionen des einen oder der mehreren Zähne entsprechen.

3. Zahnchirurgisches System nach Anspruch 1, wobei die zweite zahnchirurgische Führung (600) einen oder mehrere Hohlräume (620) aufweist, die einen Durchgang zum Bohren von Osteotomien zum Platzieren von Implantaten an der zahnchirurgischen Stelle bereitstellen.

4. Zahnchirurgisches System nach Anspruch 3, wobei die dritte zahnchirurgische Führung (700) einen oder mehrere Hohlräume (720) aufweist, die die gleichen relativen Positionen einnehmen wie der eine oder die mehreren Hohlräume der zweiten zahnchirurgischen Führung (600).

5. Zahnchirurgisches System nach Anspruch 1, wobei die dritte zahnchirurgische Führung (700) ein oder mehrere Merkmale aufweist, die natürlichen Zähnen ähneln.

6. Zahnchirurgisches System nach Anspruch 1, wobei die eine oder die mehreren Verankerungsstellen (510, 610, 710) dafür konfiguriert sind, einen Kanal zum Durchführen eines Befestigungselements durch jede unter der ersten, der zweiten und der dritten zahnchirurgischen Führung (500, 600, 700) bereitzustellen.

7. Zahnchirurgisches System nach Anspruch 1, wobei die erste zahnchirurgische Führung (500) eine oder mehrere Knochenreduktionsstellen (505) aufweist, die dafür konfiguriert sind, einen Durchgang für einen Bohrer bereitzustellen.

8. Zahnchirurgisches System nach Anspruch 7, wobei die eine oder die mehreren Knochenreduktionsstellen (505) eine lineare Anordnung von mindestens drei Kanälen aufweisen, die horizontal entlang der ersten zahnchirurgischen Führung (500) angeordnet sind.

9. Zahnchirurgisches System nach Anspruch 8, wobei die mindestens drei Kanäle eine Schablone für mindestens drei Osteotomien bereitstellen, die eine Grenze zum Reduzieren von Knochen im Kiefer abbilden.

10. Zahnchirurgisches System nach Anspruch 1, wobei mindestens eine der Verankerungsstellen (610) auf der zweiten zahnchirurgischen Führung (600) ein Kanal ist, der dafür konfiguriert ist, die zweite zahnchirurgische Führung (600) mit einem an der zahnchirurgischen Stelle angeordneten Referenzbefestigungselement auszurichten und darüber zu platzieren.

11. Zahnchirurgisches System nach Anspruch 10, wobei der Kanal mit einem unteren Rand der zweiten zahnchirurgischen Führung (600) kommuniziert.

## Revendications

1. Système de chirurgie dentaire pour implanter une prothèse, comprenant :
un premier guide chirurgical dentaire (500), un deuxième guide chirurgical dentaire (600) et un troisième guide chirurgical dentaire (700), chacun étant conçu pour s'adapter à un site chirurgical dentaire d'un patient, où le site chirurgical dentaire est une partie d'un maxillaire partiellement édenté de la mâchoire mandibulaire du patient, dans lequel :
le premier guide chirurgical dentaire (500) est conçu pour s'aligner avec une ou plusieurs dents du patient au niveau du site chirurgical ;
le deuxième guide chirurgical dentaire (600) est conçu pour permettre de percer des ostéotomies pour la mise en place d'implants au niveau du site chirurgical ;
le troisième guide chirurgical dentaire (700) est conçu pour procurer l'apparence de dents naturelles de sorte que le troisième guide chirurgical (700) peut servir de prothèse dentaire ; et
le premier guide chirurgical dentaire (500), le deuxième guide chirurgical dentaire (600) et le troisième guide chirurgical (700) comprennent chacun un ou plusieurs sites d'ancrage (510, 610, 710) pour la fixation sur l'os de mâchoire du patient, où les sites d'ancrage (510, 610, 710) occupent les mêmes positions relatives sur les premier, deuxième et troisième guides chirurgicaux dentaires (500, 600, 700) de sorte que les guides (500, 600, 700) peuvent être alignés et fixés par un seul ensemble d'ostéotomies déterminées par les sites d'ancrage (510, 610, 710) du premier guide chirurgical dentaire (500).

2. Système de chirurgie dentaire selon la revendication 1, dans lequel le premier guide chirurgical dentaire (500) comprend un ou plusieurs vides (530) correspondant aux positions d'une ou de plusieurs dents.

3. Système de chirurgie dentaire selon la revendication 1, dans lequel le deuxième guide chirurgical dentaire (600) comprend un ou plusieurs vides (620) qui procurent un passage pour le perçage d'ostéotomies permettant la mise en place d'implants au niveau d'un site chirurgical dentaire.

4. Système de chirurgie dentaire selon la revendication 3, dans lequel le troisième guide chirurgical (700) comprend un ou plusieurs vides (720) qui occupent les mêmes positions relatives que le vide ou les vides du deuxième guide chirurgical dentaire (600).

5. Système de chirurgie dentaire selon la revendication 1, dans lequel le troisième guide chirurgical dentaire (700) comprend une ou plusieurs fonctionnalités semblables à des dents naturelles.

6. Système de chirurgie dentaire selon la revendication 1, dans lequel le site ou les sites d'ancrage (510, 610, 710) sont conçus pour procurer un canal permettant le passage d'un fixateur à travers chacun parmi les premier, deuxième et troisième guides chirurgicaux dentaires (500, 600, 700).

7. Système de chirurgie dentaire selon la revendication 1, dans lequel le premier guide chirurgical dentaire (500) comprend un ou plusieurs sites de réduction osseuse (505) conçus pour fournir le passage à un foret.

8. Système de chirurgie dentaire selon la revendication 7, dans lequel le ou les sites de réduction osseuse (505) comprennent un réseau linéaire d'au moins trois canaux positionnés horizontalement le long du premier guide chirurgical dentaire (500).

9. Système de chirurgie dentaire selon la revendication 8, dans lequel les au moins trois canaux fournissent un gabarit pour au moins trois ostéotomies qui correspondent à une limite pour réduire l'os dans la mâchoire.

10. Système de chirurgie dentaire selon la revendication 1, dans lequel au moins un des sites d'ancrage (610) sur le deuxième guide chirurgical dentaire (600) est un canal conçu pour s'aligner avec le deuxième guide chirurgical (600) et s'y placer par-dessus un fixateur de référence placé au site chirurgical.

11. Système de chirurgie dentaire selon la revendication 10, dans lequel le canal est en communication avec un bord inférieur du deuxième guide chirurgical (600).
